# EUROPEAN PATENT SPECIFICATION

(11) **EP 4 341 948 B1**
(45) Date of publication and mention of the grant of the patent: **06.05.2026**
(21) Application number: 23730921.6
(22) Date of filing: 17.05.2023
(51) Int. Cl.: G16H 20/40, G16H 40/63

(54) **ADAPTED AUTONOMY FUNCTIONS AND SYSTEM INTERCONNECTIONS**
ANGEPASSTE AUTONOMIEFUNKTIONEN UND SYSTEMVERBINDUNGEN
FONCTIONS D'AUTONOMIE ADAPTÉES ET INTERCONNEXIONS DE SYSTÈME

(30) Priority: 18.05.2022 US 202217747733
(43) Date of publication of application: 27.03.2024
(73) Proprietor: Cilag GmbH International, 6300 Zug (CH)
(72) Inventor: SHELTON, IV, Frederick E., Cincinnati, Ohio 45242 (US); ROSS, Nicholas James, Cincinnati, Ohio 45242 (US); MAPLES, Curtis Anthony, Cincinnati, Ohio 45242 (US); FIEBIG, Kevin M., Cincinnati, Ohio 45242 (US); ADAMS, Shane R., Cincinnati, Ohio 45242 (US)
(74) Representative: Carpmaels & Ransford LLP
(86) International application number: PCT/IB2023/055062
(87) International publication number: WO 2023/223225

(56) References cited:
- US-A1- 2021 322 017

## Description

### Background

Surgical procedures are typically performed in surgical operating theaters or rooms in a healthcare facility such as, for example, a hospital. Various surgical devices and systems are utilized in performance of a surgical procedure. In the digital and information age, medical systems and facilities are often slower to implement systems or procedures utilizing newer and improved technologies due to patient safety and a general desire for maintaining traditional practices.

US2021/322017A1 describes a surgical system that includes a first modular device and a surgical hub, including a control circuit configured to receive first perioperative data from a second modular device; anonymize the first perioperative data; receives second perioperative data from a third modular device; anonymize the second perioperative data; and adjust at least one setting of the first modular device based on contextual information derived from the anonymized first and second perioperative data.

### Summary

Systems, methods, and instrumentalities are described herein for adapted autonomy functions and system interconnections. A surgical hub may detect a first surgical device in the surgical environment. The first surgical device may include a first plurality of features. The surgical hub may include a plurality of resources. The surgical hub may detect a second surgical device in the surgical environment. The second surgical device may include a second plurality of features. The surgical hub may determine one or more features from the first plurality of features for the first surgical device to perform and one or more features from the second plurality of features for the second surgical device to perform based on the plurality of resources.

A surgical hub is described for autonomous determination of surgical device features to be enabled for a surgical procedure. The surgical hub comprises a processor. The processor is configured to detect a first surgical device in the surgical environment. The first surgical device comprises a first plurality of features. The surgical hub comprises a plurality of resources. The processor is further configured to detect a second surgical device in the surgical environment, wherein the second surgical device comprises a second plurality of features. The processor is further configured to receive or derive an indication of the surgical procedure to be performed, the surgical procedure comprising a plurality of surgical tasks. The processor is further configured to determine one or more features from the first plurality of features for the first surgical device to be enabled to perform a surgical task of the plurality of surgical tasks and determine one or more features from the second plurality of features for the second surgical device to be enabled to perform a surgical task of the plurality of surgical tasks. The determination of which features to enable is based on the plurality of resources. The plurality of resources of the hub is finite, and the enabled instrument features require the use of the hub's resources in order to perform one or more surgical tasks. The hub advantageously autonomously coordinates the allocation of the finite hub resources to instrument features needed to perform one or more tasks of the surgical procedure to enable those instrument features. The plurality of resources may comprise computer resources, which may include power and bandwidth. A feature may be associated with a resource range (e.g. power and/or bandwidth range) at which the surgical instrument is able to perform the surgical task autonomously. In other words, the resources can be the computer processing requirements for a feature to perform a surgical task autonomously.

The processor is further configured to: send an indication of the determined one or more features to a user of the first surgical device and a user of the second surgical device; or transmit a signal to the first and/or second device to enable the determined one or more features. Advantageously, as well as determining the features to be enabled, the hub is able to transmit a signal to the first and second devices to enable the determined features, thereby preparing the devices and their features for the surgical procedure or a task of that surgical procedure (since the enablement of features can be dynamic based on the stage in the procedure). Alternatively, the hub can transmit a message to a user of the devices indicating which features are to be enabled.

Sn enabled feature may be a feature for performing the surgical task autonomously. The surgical hub may be configured to determine which tasks are to be performed autonomously by the instrument features and which are to be performed manually by a surgeon, with the allocation of resources being a factor for determining which tasks should be performed autonomously or manually and thus which features should be enabled or disabled.

The features may be features that the device is capable of performing.

The first and/or the second device may be an endocutter and the first and/or second plurality of features may comprise one or more of the following: controlling an energy source, cutting, stapling, knob orientation, body orientation, body position, clamping, anvil jaw force, reload alignment slot management.

The processor may be further configured to receive or determine an indication of the first and second plurality of features. A user may manually input the features of the devices. Alternatively, or in addition, the hub may be able to determine the features by knowing the devices. For example, a device may couple with the hub and share an identifier which identifies that device and, through this identifier, the hub may be able to determine the features of the device (for example via a lookup table stored in a memory or via an external memory/server).

The processor may be configured to detect the first and second devices when the first and second devices send a request to couple with the hub, or when they couple to the hub.

The surgical hub may comprise a plurality of ports. The first surgical device may link to the surgical hub via a first port from the plurality of ports. The second surgical device may link to the surgical hub via a second port form the plurality of ports.

Determining which features should be enabled may comprise using optimization associated with one or more rules.

The optimization associated with one or more rules may be based on at least one of: surgical context, type of the first plurality of features, type of second plurality of features, historical data, or the plurality of resources. Some features may be given a higher priority, for example based on the complexity of the task that is being performed by those features or based on the surgical outcomes of the surgical tasks when performed manually versus automatically (which could be based on historical data from past procedures including that task performed by that feature). For example, the historical data may show that the surgical outcome of a surgical task is improved when a task is performed autonomously by a feature, and the optimization may therefore prioritize the enablement of that feature to autonomously perform the surgical task. By optimizing in this way, the outcome of the surgical procedures may in general be improved. As an example, the outcome for tissue resection could correspond to the integrity of the seal line, with a seal which leaks less being equated to a more positive outcome than one which leaks more. The outcome of a procedure could also correspond to the number of complications that occur during surgery, which could include, for example, instrument malfunction, seal line leak, misfiring of a staple line, etc.

The processor may be further configured to detect a third surgical device in the surgical environment. The third surgical device may comprise a third plurality of features. The processor may be further configured to adjust the one or more features from the first plurality of features for the first surgical device to perform and the one or more features from the second plurality of features for the second surgical device to perform based on the plurality of resources and the third plurality of features. Advantageously, the surgical hub can dynamically determine which features of the devices should be enabled or disabled as the surgical procedure progresses and/or as devices are brought into the surgical environment and/or connected to the hub.

The adjustment may comprise disabling a feature that was previously enabled. By dynamically enabling and disabling features, it is possible to reallocate resources as new devices are detected or as a surgical procedure progresses.

The processor may be further configured to detect a third surgical device in the surgical environment. The third surgical device may comprise a third plurality of features. The processor may be further configured to determine one or more features from the third plurality of features for the third surgical device to perform based on the plurality of resources.

The processor may be further configured to determine one or more optional features from the first plurality of features for the first surgical device to perform and one or more optional features from the second plurality of features for the second surgical device to perform based on the plurality of resources.

The optional features may be selected by a user of the first surgical instrument and a user of the second surgical instrument.

A method is described for autonomous determination of surgical device features to be enabled for a surgical procedure. The method comprises detecting a first surgical device in the surgical environment. The first surgical device comprises a first plurality of features. A surgical hub comprises a plurality of resources. The method further comprises detecting a second surgical device in the surgical environment. The second surgical device comprises a second plurality of features. The method further comprises receiving or deriving an indication of the surgical procedure to be performed, the surgical procedure comprising a plurality of surgical tasks. The method further comprises determining one or more features from the first plurality of features for the first surgical device to be enabled to perform a surgical task of the plurality of surgical tasks and determining one or more features from the second plurality of features for the second surgical device to be enabled to perform a surgical task of the plurality of surgical tasks, wherein the determination is based on the plurality of resources. The plurality of resources of the hub may be finite, and the enabled instrument features require the use of the hub's resources in order to perform one or more surgical tasks. The method advantageously autonomously coordinates the allocation of the finite hub resources to instrument features needed to perform one or more tasks of the surgical procedure to enable those instrument features. The plurality of resources may comprise computer resources, which may include power and bandwidth. A feature may be associated with a resource range (e.g. power and/or bandwidth range) at which the surgical instrument is able to perform the surgical task autonomously. In other words, the resources can be the computer processing requirements for a feature to perform a surgical task autonomously.

The method may further comprise: sending an indication of the determined one or more features to a user of the first surgical device and a user of the second surgical device; or transmitting a signal to the first and/or second device to enable the determined one or more features. Advantageously, as well as determining the features to be enabled, the method comprises transmitting a signal to the first and second devices to enable the determined features, thereby preparing the devices and their features for the surgical procedure or a task of that surgical procedure (since the enablement of features can be dynamic based on the stage in the procedure). Alternatively, the method comprises transmitting a message to a user of the devices indicating which features are to be enabled.

An enabled feature may be a feature for performing the surgical task autonomously. The method is configured to determine which tasks are to be performed autonomously by the instrument features and which are to be performed manually by a surgeon, with the allocation of resources being a factor for determining which tasks should be performed autonomously or manually and thus which features should be enabled or disabled.

The features may be features that the device is capable of performing.

The first and/or the second device may be an endocutter and the plurality of first and/or second features may comprise one or more of the following: controlling an energy source, cutting, stapling, knob orientation, body orientation, body position, clamping, anvil jaw force, reload alignment slot management.

The method may further comprise receiving or determining an indication of the first and second plurality of features.

Detecting the first and second devices may comprise detecting when the first and second devices send a request to couple with the hub, or detecting when they have been coupled to the hub.

The surgical hub may comprise a plurality of ports. The first surgical device may link to the surgical hub via a first port from the plurality of ports. The second surgical device may link to the surgical hub via a second port form the plurality of ports.

Determining which features should be enabled may comprise using optimization associated with one or more rules.

The optimization associated with one or more rules may be based on at least one of: surgical context, type of the first plurality of features, type of second plurality of features, historical data, or the plurality of resources. Some features may be given a higher priority, for example based on the complexity of the task that is being performed by those features or based on the surgical outcomes of the surgical tasks when performed manually versus automatically (which could be based on historical data from past procedures including that task performed by that feature). For example, the historical data may show that the surgical outcome of a surgical task is improved when a task is performed autonomously by a feature, and the optimization may therefore prioritize the enablement of that feature to autonomously perform the surgical task. By optimizing in this way, the outcome of the surgical procedures may in general be improved. As an example, the outcome for tissue resection could correspond to the integrity of the seal line, with a seal which leaks less being equated to a more positive outcome than one which leaks more. The outcome of a procedure could also correspond to the number of complications that occur during surgery, which could include, for example, instrument malfunction, seal line leak, misfiring of a staple line, etc.

The method may further comprise detecting a third surgical device in the surgical environment. The third surgical device may comprise a third plurality of features. The method may further comprise adjusting the one or more features from the first plurality of features for the first surgical device to perform and the one or more features from the second plurality of features for the second surgical device to perform based on the plurality of resources and the third plurality of features. Advantageously, the method comprises dynamically determining which features of the devices should be enabled or disabled as the surgical procedure progresses and/or as devices are brought into the surgical environment and/or connected to the hub.

The adjustment may comprise disabling a feature that was previously enabled. By dynamically enabling and disabling features, it is possible to reallocate resources as new devices are detected or as a surgical procedure progresses.

The method may further comprise detecting a third surgical device in the surgical environment. The third surgical device may comprise a third plurality of features. The method may further comprise determining one or more features from the third plurality of features for the third surgical device to perform based on the plurality of resources.

The method may further comprise determining one or more optional features from the first plurality of features for the first surgical device to perform and one or more optional features from the second plurality of features for the second surgical device to perform based on the plurality of resources.

The optional features may be selected by a user of the first surgical instrument and a user of the second surgical instrument.

A computer program is described comprising instructions which, when the program is executed by a computer, cause the computer to carry out any previously described method.

A computer-readable medium is described comprising instructions which, when executed by a computer, cause the computer to carry out any previously described method.

### Brief Description of the Drawings

FIG. 1 is a block diagram of a computer-implemented surgical system.
FIG. 2 shows an example surgical system in a surgical operating room.
FIG. 3 illustrates an example surgical hub paired with various systems.
FIG. 4 illustrates a surgical data network having a set of communication surgical hubs configured to connect with a set of sensing systems, an environmental sensing system, a set of devices, etc.
FIG. 5 illustrates a logic diagram of a control system of a surgical instrument.
FIG. 6 shows an example surgical system that includes a handle having a controller and a motor, an adapter releasably coupled to the handle, and a loading unit releasably coupled to the adapter.
FIG. 7 shows an example situationally aware surgical system.
FIG. 8 shows an example of a surgical autonomous system.
FIG. 9 shows an example of an overview of an adapting interconnected surgical system.
FIG. 10 shows an example of the relationship between feature sets, surgical hub, and resource management.
FIG. 11 shows an example of a matrix generator and feature determination.
FIG. 12 shows a flow chart of an adapted interconnected surgical system.
FIG. 13 shows an example of an overview of a rules engine.

### Detailed Description

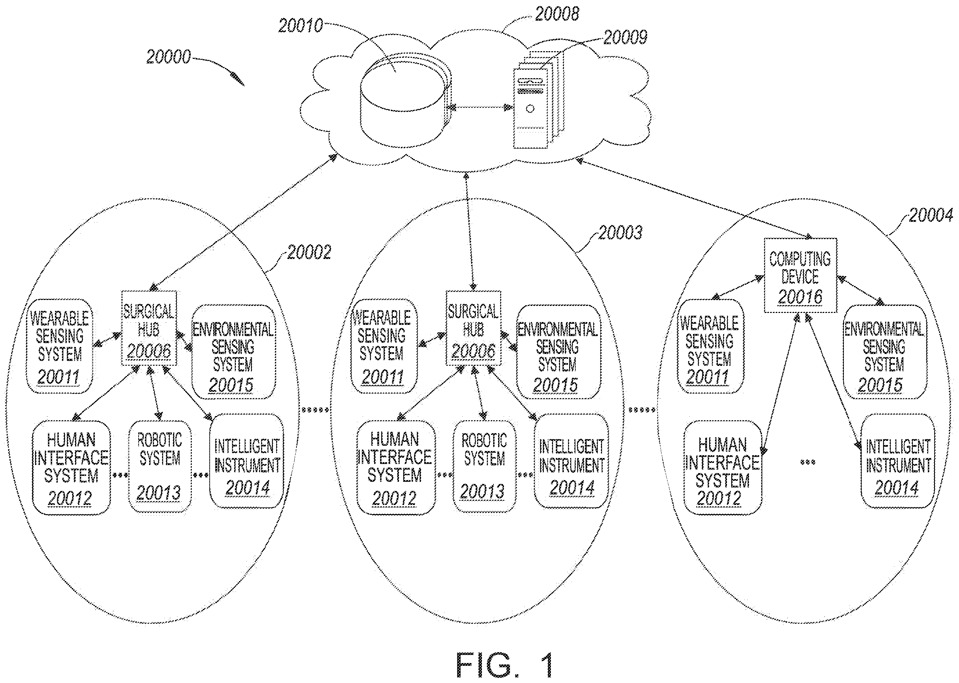

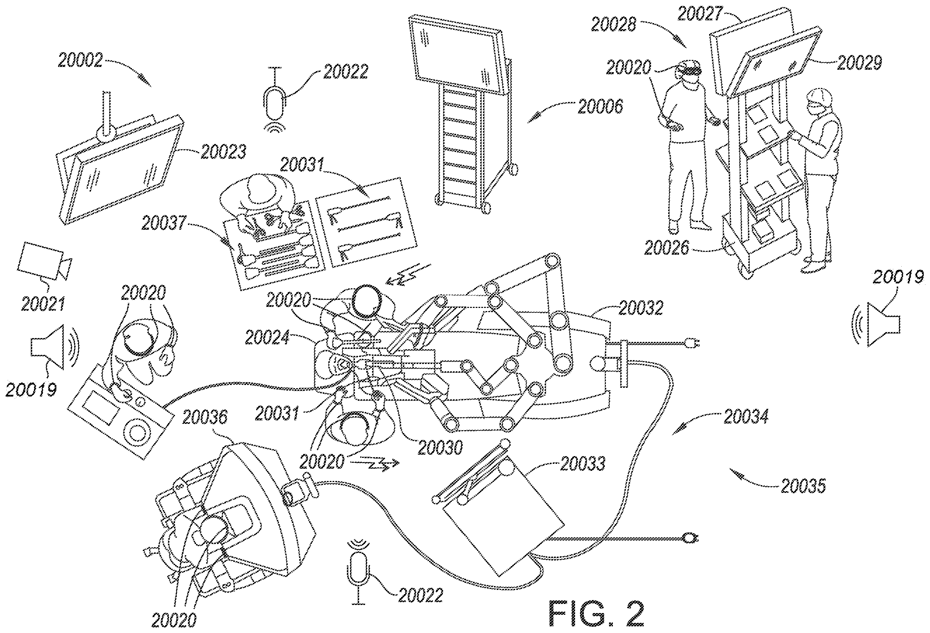

FIG. 1 is a block diagram of a computer-implemented surgical system 20000. An example surgical system such as the surgical system 20000 may include one or more surgical systems (e.g., surgical sub-systems) 20002, 20003 and 20004. For example, surgical system 20002 may include a computer-implemented interactive surgical system. For example, surgical system 20002 may include a surgical hub 20006 and/or a computing device 20016 in communication with a cloud computing system 20008, for example, as desFcribed in FIG. 2. The cloud computing system 20008 may include at least one remote cloud server 20009 and at least one remote cloud storage unit 20010. Example surgical systems 20002, 20003, or 20004 may include a wearable sensing system 20011, an environmental sensing system 20015, a robotic system 20013, one or more intelligent instruments 20014, human interface system 20012, etc. The human interface system is also referred herein as the human interface device. The wearable sensing system 20011 may include one or more HCP sensing systems, and/or one or more patient sensing systems. The environmental sensing system 20015 may include one or more devices, for example, used for measuring one or more environmental attributes, for example, as further described in FIG. 2. The robotic system 20013 may include a plurality of devices used for performing a surgical procedure, for example, as further described in FIG. 2.

The surgical system 20002 may be in communication with a remote server 20009 that may be part of a cloud computing system 20008. In an example, the surgical system 20002 may be in communication with a remote server 20009 via an internet service provider's cable/FIOS networking node. In an example, a patient sensing system may be in direct communication with a remote server 20009. The surgical system 20002 and/or a component therein may communicate with the remote servers 20009 via a cellular transmission/reception point (TRP) or a base station using one or more of the following cellular protocols: GSM/GPRS/EDGE (2G), UMTS/HSPA (3G), long term evolution (LTE) or 4G, LTE-Advanced (LTE-A), new radio (NR) or 5G.

A surgical hub 20006 may have cooperative interactions with one of more means of displaying the image from the laparoscopic scope and information from one or more other smart devices and one or more sensing systems 20011. The surgical hub 20006 may interact with one or more sensing systems 20011, one or more smart devices, and multiple displays. The surgical hub 20006 may be configured to gather measurement data from the one or more sensing systems 20011 and send notifications or control messages to the one or more sensing systems 20011. The surgical hub 20006 may send and/or receive information including notification information to and/or from the human interface system 20012. The human interface system 20012 may include one or more human interface devices (HIDs). The surgical hub 20006 may send and/or receive notification information or control information to audio, display and/or control information to various devices that are in communication with the surgical hub.

For example, the sensing systems 20001 may include the wearable sensing system 20011 (which may include one or more HCP sensing systems and one or more patient sensing systems) and the environmental sensing system 20015 as discussed in FIG. 1. The one or more sensing systems 20001 may measure data relating to various biomarkers. The one or more sensing systems 20001 may measure the biomarkers using one or more sensors, for example, photosensors (e.g., photodiodes, photoresistors), mechanical sensors (e.g., motion sensors), acoustic sensors, electrical sensors, electrochemical sensors, thermoelectric sensors, infrared sensors, etc. The one or more sensors may measure the biomarkers as described herein using one of more of the following sensing technologies: photoplethysmography, electrocardiography, electroencephalography, colorimetry, impedimentary, potentiometry, amperometry, etc.

The biomarkers measured by the one or more sensing systems 20001 may include, but are not limited to, sleep, core body temperature, maximal oxygen consumption, physical activity, alcohol consumption, respiration rate, oxygen saturation, blood pressure, blood sugar, heart rate variability, blood potential of hydrogen, hydration state, heart rate, skin conductance, peripheral temperature, tissue perfusion pressure, coughing and sneezing, gastrointestinal motility, gastrointestinal tract imaging, respiratory tract bacteria, edema, mental aspects, sweat, circulating tumor cells, autonomic tone, circadian rhythm, and/or menstrual cycle.

The biomarkers may relate to physiologic systems, which may include, but are not limited to, behavior and psychology, cardiovascular system, renal system, skin system, nervous system, gastrointestinal system, respiratory system, endocrine system, immune system, tumor, musculoskeletal system, and/or reproductive system. Information from the biomarkers may be determined and/or used by the computer-implemented patient and the surgical system 20000, for example. The information from the biomarkers may be determined and/or used by the computer-implemented patient and the surgical system 20000 to improve said systems and/or to improve patient outcomes, for example. The one or more sensing systems 20001, biomarkers 20005, and physiological systems are described in more detail in U.S. App No. 17/156,287 (attorney docket number END9290USNP1), titled METHOD OF ADJUSTING A SURGICAL PARAMETER BASED ON BIOMARKER MEASUREMENTS, filed January 22, 2021.

FIG. 2 shows an example of a surgical system 20002 in a surgical operating room. As illustrated in FIG. 2, a patient is being operated on by one or more health care professionals (HCPs). The HCPs are being monitored by one or more HCP sensing systems 20020 worn by the HCPs. The HCPs and the environment surrounding the HCPs may also be monitored by one or more environmental sensing systems including, for example, a set of cameras 20021, a set of microphones 20022, and other sensors that may be deployed in the operating room. The HCP sensing systems 20020 and the environmental sensing systems may be in communication with a surgical hub 20006, which in turn may be in communication with one or more cloud servers 20009 of the cloud computing system 20008, as shown in FIG. 1. The environmental sensing systems may be used for measuring one or more environmental attributes, for example, HCP position in the surgical theater, HCP movements, ambient noise in the surgical theater, temperature /humidity in the surgical theater, etc.

As illustrated in FIG. 2, a primary display 20023 and one or more audio output devices (e.g., speakers 20019) are positioned in the sterile field to be visible to an operator at the operating table 20024. In addition, a visualization/notification tower 20026 is positioned outside the sterile field. The visualization/notification tower 20026 may include a first non-sterile human interactive device (HID) 20027 and a second non-sterile HID 20029, which may face away from each other. The HID may be a display or a display with a touchscreen allowing a human to interface directly with the HID. A human interface system, guided by the surgical hub 20006, may be configured to utilize the HIDs 20027, 20029, and 20023 to coordinate information flow to operators inside and outside the sterile field. In an example, the surgical hub 20006 may cause an HID (e.g., the primary HID 20023) to display a notification and/or information about the patient and/or a surgical procedure step. In an example, the surgical hub 20006 may prompt for and/or receive input from personnel in the sterile field or in the non-sterile area. In an example, the surgical hub 20006 may cause an HID to display a snapshot of a surgical site, as recorded by an imaging device 20030, on a non-sterile HID 20027 or 20029, while maintaining a live feed of the surgical site on the primary HID 20023. The snapshot on the non-sterile display 20027 or 20029 can permit a non-sterile operator to perform a diagnostic step relevant to the surgical procedure, for example.

In one aspect, the surgical hub 20006 may be configured to route a diagnostic input or feedback entered by a non-sterile operator at the visualization tower 20026 to the primary display 20023 within the sterile field, where it can be viewed by a sterile operator at the operating table. In one example, the input can be in the form of a modification to the snapshot displayed on the non-sterile display 20027 or 20029, which can be routed to the primary display 20023 by the surgical hub 20006.

Referring to FIG. 2, a surgical instrument 20031 is being used in the surgical procedure as part of the surgical system 20002. The hub 20006 may be configured to coordinate information flow to a display of the surgical instrument 20031. For example, in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018. A diagnostic input or feedback entered by a non-sterile operator at the visualization tower 20026 can be routed by the hub 20006 to the surgical instrument display within the sterile field, where it can be viewed by the operator of the surgical instrument 20031. Example surgical instruments that are suitable for use with the surgical system 20002 are described under the heading "Surgical Instrument Hardware" and in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018.

FIG. 2 illustrates an example of a surgical system 20002 being used to perform a surgical procedure on a patient who is lying down on an operating table 20024 in a surgical operating room 20035. A robotic system 20034 may be used in the surgical procedure as a part of the surgical system 20002. The robotic system 20034 may include a surgeon's console 20036, a patient side cart 20032 (surgical robot), and a surgical robotic hub 20033. The patient side cart 20032 can manipulate at least one removably coupled surgical tool 20037 through a minimally invasive incision in the body of the patient while the surgeon views the surgical site through the surgeon's console 20036. An image of the surgical site can be obtained by a medical imaging device 20030, which can be manipulated by the patient side cart 20032 to orient the imaging device 20030. The robotic hub 20033 can be used to process the images of the surgical site for subsequent display to the surgeon through the surgeon's console 20036.

Other types of robotic systems can be readily adapted for use with the surgical system 20002. Various examples of robotic systems and surgical tools that are suitable for use with the present disclosure are described in U.S. Patent Application Publication No. US 2019-0201137 A1 (U.S. Patent Application No. 16/209,407), titled METHOD OF ROBOTIC HUB COMMUNICATION, DETECTION, AND CONTROL, filed December 4, 2018.

Various examples of cloud-based analytics that are performed by the cloud computing system 20008, and are suitable for use with the present disclosure, are described in U.S. Patent Application Publication No. US 2019-0206569 A1 (U.S. Patent Application No. 16/209,403), titled METHOD OF CLOUD BASED DATA ANALYTICS FOR USE WITH THE HUB, filed December 4, 2018.

In various aspects, the imaging device 20030 may include at least one image sensor and one or more optical components. Suitable image sensors may include, but are not limited to, Charge-Coupled Device (CCD) sensors and Complementary Metal-Oxide Semiconductor (CMOS) sensors.

The optical components of the imaging device 20030 may include one or more illumination sources and/or one or more lenses. The one or more illumination sources may be directed to illuminate portions of the surgical field. The one or more image sensors may receive light reflected or refracted from the surgical field, including light reflected or refracted from tissue and/or surgical instruments.

The one or more illumination sources may be configured to radiate electromagnetic energy in the visible spectrum as well as the invisible spectrum. The visible spectrum, sometimes referred to as the optical spectrum or luminous spectrum, is the portion of the electromagnetic spectrum that is visible to (i.e., can be detected by) the human eye and may be referred to as visible light or simply light. A typical human eye will respond to wavelengths in air that range from about 380 nm to about 750 nm.

The invisible spectrum (e.g., the non-luminous spectrum) is the portion of the electromagnetic spectrum that lies below and above the visible spectrum (i.e., wavelengths below about 380 nm and above about 750 nm). The invisible spectrum is not detectable by the human eye. Wavelengths greater than about 750 nm are longer than the red visible spectrum, and they become invisible infrared (IR), microwave, and radio electromagnetic radiation. Wavelengths less than about 380 nm are shorter than the violet spectrum, and they become invisible ultraviolet, x-ray, and gamma ray electromagnetic radiation.

In various aspects, the imaging device 20030 is configured for use in a minimally invasive procedure. Examples of imaging devices suitable for use with the present disclosure include, but are not limited to, an arthroscope, angioscope, bronchoscope, choledochoscope, colonoscope, cytoscope, duodenoscope, enteroscope, esophagogastro-duodenoscope (gastroscope), endoscope, laryngoscope, nasopharyngo-neproscope, sigmoidoscope, thoracoscope, and ureteroscope.

The imaging device may employ multi-spectrum monitoring to discriminate topography and underlying structures. A multi-spectral image is one that captures image data within specific wavelength ranges across the electromagnetic spectrum. The wavelengths may be separated by filters or by the use of instruments that are sensitive to particular wavelengths, including light from frequencies beyond the visible light range, e.g., IR and ultraviolet. Spectral imaging can allow extraction of additional information that the human eye fails to capture with its receptors for red, green, and blue. The use of multi-spectral imaging is described in greater detail under the heading "Advanced Imaging Acquisition Module" in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018. Multi-spectrum monitoring can be a useful tool in relocating a surgical field after a surgical task is completed to perform one or more of the previously described tests on the treated tissue. It is axiomatic that strict sterilization of the operating room and surgical equipment is required during any surgery. The strict hygiene and sterilization conditions required in a "surgical theater," i.e., an operating or treatment room, necessitate the highest possible sterility of all medical devices and equipment. Part of that sterilization process is the need to sterilize anything that comes in contact with the patient or penetrates the sterile field, including the imaging device 20030 and its attachments and components. It will be appreciated that the sterile field may be considered a specified area, such as within a tray or on a sterile towel, that is considered free of microorganisms, or the sterile field may be considered an area, immediately around a patient, who has been prepared for a surgical procedure. The sterile field may include the scrubbed team members, who are properly attired, and all furniture and fixtures in the area.

Wearable sensing system 20011 illustrated in FIG. 1 may include one or more sensing systems, for example, HCP sensing systems 20020 as shown in FIG. 2. The HCP sensing systems 20020 may include sensing systems to monitor and detect a set of physical states and/or a set of physiological states of a healthcare personnel (HCP). An HCP may be a surgeon or one or more healthcare personnel assisting the surgeon or other healthcare service providers in general. In an example, a sensing system 20020 may measure a set of biomarkers to monitor the heart rate of an HCP. In an example, a sensing system 20020 worn on a surgeon's wrist (e.g., a watch or a wristband) may use an accelerometer to detect hand motion and/or shakes and determine the magnitude and frequency of tremors. The sensing system 20020 may send the measurement data associated with the set of biomarkers and the data associated with a physical state of the surgeon to the surgical hub 20006 for further processing. One or more environmental sensing devices may send environmental information to the surgical hub 20006. For example, the environmental sensing devices may include a camera 20021 for detecting hand/body position of an HCP. The environmental sensing devices may include microphones 20022 for measuring the ambient noise in the surgical theater. Other environmental sensing devices may include devices, for example, a thermometer to measure temperature and a hygrometer to measure humidity of the surroundings in the surgical theater, etc. The surgical hub 20006, alone or in communication with the cloud computing system, may use the surgeon biomarker measurement data and/or environmental sensing information to modify the control algorithms of hand-held instruments or the averaging delay of a robotic interface, for example, to minimize tremors. In an example, the HCP sensing systems 20020 may measure one or more surgeon biomarkers associated with an HCP and send the measurement data associated with the surgeon biomarkers to the surgical hub 20006. The HCP sensing systems 20020 may use one or more of the following RF protocols for communicating with the surgical hub 20006: Bluetooth, Bluetooth Low-Energy (BLE), Bluetooth Smart, Zigbee, Z-wave, IPv6 Low-power wireless Personal Area Network (6LoWPAN), Wi-Fi. The surgeon biomarkers may include one or more of the following: stress, heart rate, etc. The environmental measurements from the surgical theater may include ambient noise level associated with the surgeon or the patient, surgeon and/or staff movements, surgeon and/or staff attention level, etc.

The surgical hub 20006 may use the surgeon biomarker measurement data associated with an HCP to adaptively control one or more surgical instruments 20031. For example, the surgical hub 20006 may send a control program to a surgical instrument 20031 to control its actuators to limit or compensate for fatigue and use of fine motor skills. The surgical hub 20006 may send the control program based on situational awareness and/or the context on importance or criticality of a task. The control program may instruct the instrument to alter operation to provide more control when control is needed.

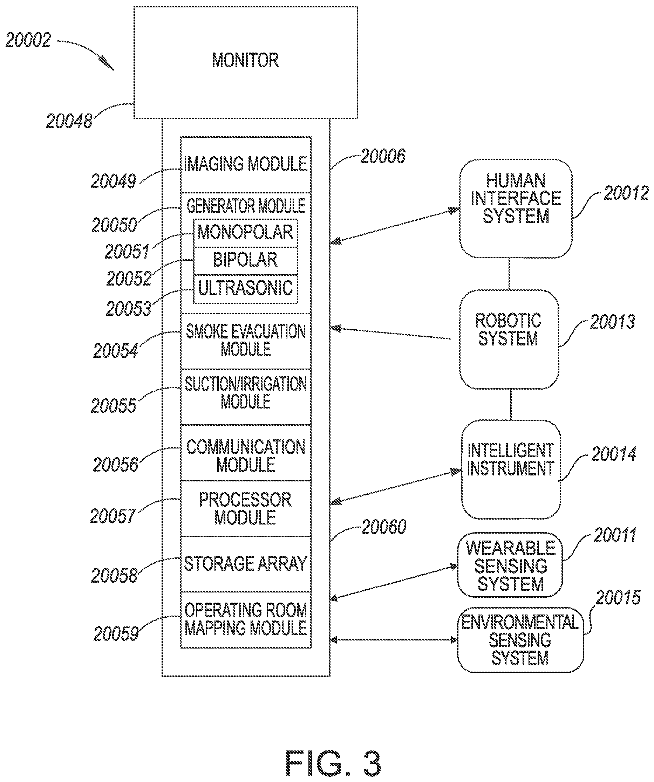

FIG. 3 shows an example surgical system 20002 with a surgical hub 20006. The surgical hub 20006 may be paired with, via a modular control, a wearable sensing system 20011, an environmental sensing system 20015, a human interface system 20012, a robotic system 20013, and an intelligent instrument 20014. The hub 20006 includes a display 20048, an imaging module 20049, a generator module 20050, a communication module 20056, a processor module 20057, a storage array 20058, and an operating-room mapping module 20059. In certain aspects, as illustrated in FIG. 3, the hub 20006 further includes a smoke evacuation module 20054 and/or a suction/irrigation module 20055. The various modules and systems may be connected to the modular control either directly via a router or via the communication module 20056. The operating theater devices may be coupled to cloud computing resources and data storage via the modular control. The human interface system 20012 may include a display sub-system and a notification sub-system.

The modular control may be coupled to non-contact sensor module. The non-contact sensor module may measure the dimensions of the operating theater and generate a map of the surgical theater using, ultrasonic, laser-type, and/or the like, non-contact measurement devices. Other distance sensors can be employed to determine the bounds of an operating room. An ultrasound-based non-contact sensor module may scan the operating theater by transmitting a burst of ultrasound and receiving the echo when it bounces off the perimeter walls of an operating theater as described under the heading "Surgical Hub Spatial Awareness Within an Operating Room" in U.S. Provisional Patent Application Serial No. 62/611,341, titled INTERACTIVE SURGICAL PLATFORM, filed December 28, 2017. The sensor module may be configured to determine the size of the operating theater and to adjust Bluetooth-pairing distance limits. A laser-based non-contact sensor module may scan the operating theater by transmitting laser light pulses, receiving laser light pulses that bounce off the perimeter walls of the operating theater, and comparing the phase of the transmitted pulse to the received pulse to determine the size of the operating theater and to adjust Bluetooth pairing distance limits, for example.

During a surgical procedure, energy application to tissue, for sealing and/or cutting, is generally associated with smoke evacuation, suction of excess fluid, and/or irrigation of the tissue. Fluid, power, and/or data lines from different sources are often entangled during the surgical procedure. Valuable time can be lost addressing this issue during a surgical procedure. Detangling the lines may necessitate disconnecting the lines from their respective modules, which may require resetting the modules. The hub modular enclosure 20060 offers a unified environment for managing the power, data, and fluid lines, which reduces the frequency of entanglement between such lines. Aspects of the present disclosure present a surgical hub 20006 for use in a surgical procedure that involves energy application to tissue at a surgical site. The surgical hub 20006 includes a hub enclosure 20060 and a combo generator module slidably receivable in a docking station of the hub enclosure 20060. The docking station includes data and power contacts. The combo generator module includes two or more of an ultrasonic energy generator component, a bipolar RF energy generator component, and a monopolar RF energy generator component that are housed in a single unit. In one aspect, the combo generator module also includes a smoke evacuation component, at least one energy delivery cable for connecting the combo generator module to a surgical instrument, at least one smoke evacuation component configured to evacuate smoke, fluid, and/or particulates generated by the application of therapeutic energy to the tissue, and a fluid line extending from the remote surgical site to the smoke evacuation component. In one aspect, the fluid line may be a first fluid line, and a second fluid line may extend from the remote surgical site to a suction and irrigation module 20055 slidably received in the hub enclosure 20060. In one aspect, the hub enclosure 20060 may include a fluid interface. Certain surgical procedures may require the application of more than one energy type to the tissue. One energy type may be more beneficial for cutting the tissue, while another different energy type may be more beneficial for sealing the tissue. For example, a bipolar generator can be used to seal the tissue while an ultrasonic generator can be used to cut the sealed tissue. Aspects of the present disclosure present a solution where a hub modular enclosure 20060 is configured to accommodate different generators and facilitate an interactive communication therebetween. One of the advantages of the hub modular enclosure 20060 is enabling the quick removal and/or replacement of various modules. Aspects of the present disclosure present a modular surgical enclosure for use in a surgical procedure that involves energy application to tissue. The modular surgical enclosure includes a first energy-generator module, configured to generate a first energy for application to the tissue, and a first docking station comprising a first docking port that includes first data and power contacts, wherein the first energy-generator module is slidably movable into an electrical engagement with the power and data contacts and wherein the first energy-generator module is slidably movable out of the electrical engagement with the first power and data contacts. Further to the above, the modular surgical enclosure also includes a second energy-generator module configured to generate a second energy, different than the first energy, for application to the tissue, and a second docking station comprising a second docking port that includes second data and power contacts, wherein the second energy generator module is slidably movable into an electrical engagement with the power and data contacts, and wherein the second energy-generator module is slidably movable out of the electrical engagement with the second power and data contacts. In addition, the modular surgical enclosure also includes a communication bus between the first docking port and the second docking port, configured to facilitate communication between the first energy-generator module and the second energy-generator module. Referring to FIG. 3, aspects of the present disclosure are presented for a hub modular enclosure 20060 that allows the modular integration of a generator module 20050, a smoke evacuation module 20054, and a suction/irrigation module 20055. The hub modular enclosure 20060 further facilitates interactive communication between the modules 20059, 20054, and 20055. The generator module 20050 can be with integrated monopolar, bipolar, and ultrasonic components supported in a single housing unit slidably insertable into the hub modular enclosure 20060. The generator module 20050 can be configured to connect to a monopolar device 20051, a bipolar device 20052, and an ultrasonic device 20053. Alternatively, the generator module 20050 may comprise a series of monopolar, bipolar, and/or ultrasonic generator modules that interact through the hub modular enclosure 20060. The hub modular enclosure 20060 can be configured to facilitate the insertion of multiple generators and interactive communication between the generators docked into the hub modular enclosure 20060 so that the generators would act as a single generator.

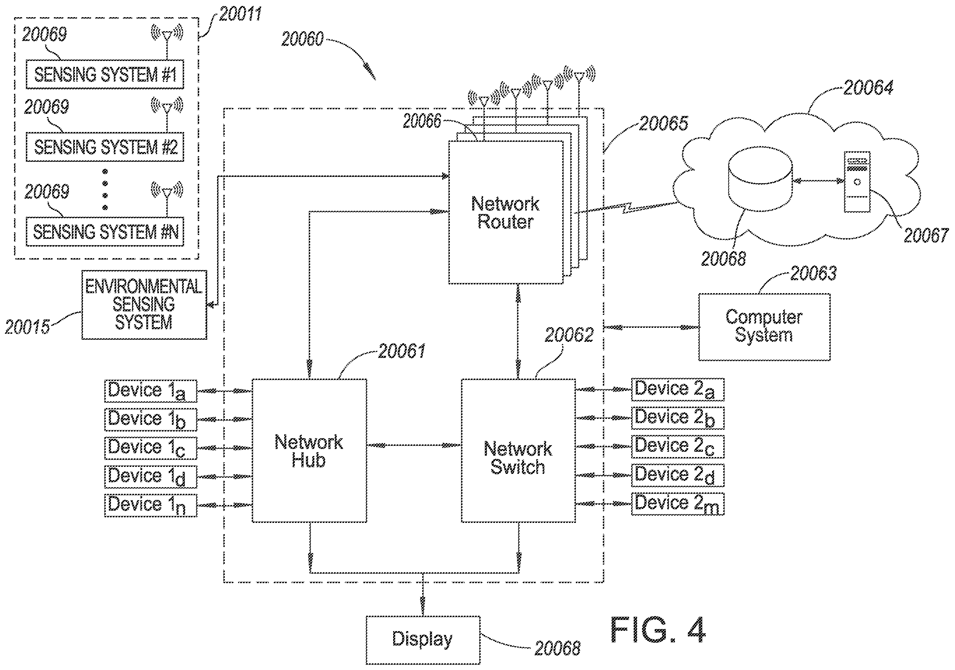

FIG. 4 illustrates a surgical data network having a set of communication hubs configured to connect a set of sensing systems, environment sensing system(s), and a set of other modular devices located in one or more operating theaters of a healthcare facility, a patient recovery room, or a room in a healthcare facility specially equipped for surgical operations, to the cloud, in accordance with at least one aspect of the present disclosure.

As illustrated in FIG. 4, a surgical hub system 20060 may include a modular communication hub 20065 that is configured to connect modular devices located in a healthcare facility to a cloud-based system (e.g., a cloud computing system 20064 that may include a remote server 20067 coupled to a remote storage 20068). The modular communication hub 20065 and the devices may be connected in a room in a healthcare facility specially equipped for surgical operations. In one aspect, the modular communication hub 20065 may include a network hub 20061 and/or a network switch 20062 in communication with a network router 20066. The modular communication hub 20065 may be coupled to a local computer system 20063 to provide local computer processing and data manipulation.

The computer system 20063 may comprise a processor and a network interface 20100. The processor may be coupled to a communication module, storage, memory, non-volatile memory, and input/output (I/O) interface via a system bus. The system bus can be any of several types of bus structure(s) including the memory bus or memory controller, a peripheral bus or external bus, and/or a local bus using any variety of available bus architectures including, but not limited to, 9-bit bus, Industrial Standard Architecture (ISA), Micro-Charmel Architecture (MSA), Extended ISA (EISA), Intelligent Drive Electronics (IDE), VESA Local Bus (VLB), Peripheral Component Interconnect (PCI), USB, Advanced Graphics Port (AGP), Personal Computer Memory Card International Association bus (PCMCIA), Small Computer Systems Interface (SCSI), or any other proprietary bus.

The processor may be any single-core or multicore processor such as those known under the trade name ARM Cortex by Texas Instruments. In one aspect, the processor may be an LM4F230H5QR ARM Cortex-M4F Processor Core, available from Texas Instruments, for example, comprising an on-chip memory of 256 KB single-cycle flash memory, or other non-volatile memory, up to 40 MHz, a prefetch buffer to improve performance above 40 MHz, a 32 KB single-cycle serial random access memory (SRAM), an internal read-only memory (ROM) loaded with StellarisWare^{®} software, a 2 KB electrically erasable programmable read-only memory (EEPROM), and/or one or more pulse width modulation (PWM) modules, one or more quadrature encoder inputs (QEI) analogs, one or more 12-bit analog-to-digital converters (ADCs) with 12 analog input channels, details of which are available for the product datasheet.

In an example, the processor may comprise a safety controller comprising two controller-based families such as TMS570 and RM4x, known under the trade name Hercules ARM Cortex R4, also by Texas Instruments. The safety controller may be configured specifically for IEC 61508 and ISO 26262 safety critical applications, among others, to provide advanced integrated safety features while delivering scalable performance, connectivity, and memory options.

It is to be appreciated that the computer system 20063 may include software that acts as an intermediary between users and the basic computer resources described in a suitable operating environment. Such software may include an operating system. The operating system, which can be stored on the disk storage, may act to control and allocate resources of the computer system. System applications may take advantage of the management of resources by the operating system through program modules and program data stored either in the system memory or on the disk storage. It is to be appreciated that various components described herein can be implemented with various operating systems or combinations of operating systems.

A user may enter commands or information into the computer system 20063 through input device(s) coupled to the I/O interface. The input devices may include, but are not limited to, a pointing device such as a mouse, trackball, stylus, touch pad, keyboard, microphone, joystick, game pad, satellite dish, scanner, TV tuner card, digital camera, digital video camera, web camera, and the like. These and other input devices connect to the processor 20102 through the system bus via interface port(s). The interface port(s) include, for example, a serial port, a parallel port, a game port, and a USB. The output device(s) use some of the same types of ports as input device(s). Thus, for example, a USB port may be used to provide input to the computer system 20063 and to output information from the computer system 20063 to an output device. An output adapter may be provided to illustrate that there can be some output devices like monitors, displays, speakers, and printers, among other output devices that may require special adapters. The output adapters may include, by way of illustration and not limitation, video and sound cards that provide a means of connection between the output device and the system bus. It should be noted that other devices and/or systems of devices, such as remote computer(s), may provide both input and output capabilities.

The computer system 20063 can operate in a networked environment using logical connections to one or more remote computers, such as cloud computer(s), or local computers. The remote cloud computer(s) can be a personal computer, server, router, network PC, workstation, microprocessor-based appliance, peer device, or other common network node, and the like, and typically includes many or all of the elements described relative to the computer system. For purposes of brevity, only a memory storage device is illustrated with the remote computer(s). The remote computer(s) may be logically connected to the computer system through a network interface and then physically connected via a communication connection. The network interface may encompass communication networks such as local area networks (LANs) and wide area networks (WANs). LAN technologies may include Fiber Distributed Data Interface (FDDI), Copper Distributed Data Interface (CDDI), Ethernet/IEEE 802.3, Token Ring/IEEE 802.5, and the like. WAN technologies may include, but are not limited to, point-to-point links, circuit-switching networks like Integrated Services Digital Networks (ISDN) and variations thereon, packet-switching networks, and Digital Subscriber Lines (DSL).

In various examples, the computer system 20063 may comprise an image processor, image-processing engine, media processor, or any specialized digital signal processor (DSP) used for the processing of digital images. The image processor may employ parallel computing with single instruction, multiple data (SIMD) or multiple instruction, multiple data (MIMD) technologies to increase speed and efficiency. The digital image-processing engine can perform a range of tasks. The image processor may be a system on a chip with multicore processor architecture.

The communication connection(s) may refer to the hardware/software employed to connect the network interface to the bus. While the communication connection is shown for illustrative clarity inside the computer system 20063, it can also be external to the computer system 20063. The hardware/software necessary for connection to the network interface may include, for illustrative purposes only, internal and external technologies such as modems, including regular telephone-grade modems, cable modems, optical fiber modems, and DSL modems, ISDN adapters, and Ethernet cards. In some examples, the network interface may also be provided using an RF interface.

Surgical data network associated with the surgical hub system 20060 may be configured as passive, intelligent, or switching. A passive surgical data network serves as a conduit for the data, enabling it to go from one device (or segment) to another and to the cloud computing resources. An intelligent surgical data network includes additional features to enable the traffic passing through the surgical data network to be monitored and to configure each port in the network hub 20061 or network switch 20062. An intelligent surgical data network may be referred to as a manageable hub or switch. A switching hub reads the destination address of each packet and then forwards the packet to the correct port.

Modular devices 1a-1n located in the operating theater may be coupled to the modular communication hub 20065. The network hub 20061 and/or the network switch 20062 may be coupled to a network router 20066 to connect the devices 1a-1n to the cloud computing system 20064 or the local computer system 20063. Data associated with the devices 1a-1n may be transferred to cloud-based computers via the router for remote data processing and manipulation. Data associated with the devices 1a-1n may also be transferred to the local computer system 20063 for local data processing and manipulation. Modular devices 2a-2m located in the same operating theater also may be coupled to a network switch 20062. The network switch 20062 may be coupled to the network hub 20061 and/or the network router 20066 to connect the devices 2a-2m to the cloud 20064. Data associated with the devices 2a-2m may be transferred to the cloud computing system 20064 via the network router 20066 for data processing and manipulation. Data associated with the devices 2a-2m may also be transferred to the local computer system 20063 for local data processing and manipulation.

The wearable sensing system 20011 may include one or more sensing systems 20069. The sensing systems 20069 may include an HCP sensing system and/or a patient sensing system. The one or more sensing systems 20069 may be in communication with the computer system 20063 of a surgical hub system 20060 or the cloud server 20067 directly via one of the network routers 20066 or via a network hub 20061 or network switching 20062 that is in communication with the network routers 20066.

The sensing systems 20069 may be coupled to the network router 20066 to connect to the sensing systems 20069 to the local computer system 20063 and/or the cloud computing system 20064. Data associated with the sensing systems 20069 may be transferred to the cloud computing system 20064 via the network router 20066 for data processing and manipulation. Data associated with the sensing systems 20069 may also be transferred to the local computer system 20063 for local data processing and manipulation.

As illustrated in FIG. 4, the surgical hub system 20060 may be expanded by interconnecting multiple network hubs 20061 and/or multiple network switches 20062 with multiple network routers 20066. The modular communication hub 20065 may be contained in a modular control tower configured to receive multiple devices 1a-1n/2a-2m. The local computer system 20063 also may be contained in a modular control tower. The modular communication hub 20065 may be connected to a display 20068 to display images obtained by some of the devices 1a-1n/2a-2m, for example during surgical procedures. In various aspects, the devices 1a-1n/2a-2m may include, for example, various modules such as an imaging module coupled to an endoscope, a generator module coupled to an energy-based surgical device, a smoke evacuation module, a suction/irrigation module, a communication module, a processor module, a storage array, a surgical device coupled to a display, and/or a non-contact sensor module, among other modular devices that may be connected to the modular communication hub 20065 of the surgical data network.

In one aspect, the surgical hub system 20060 illustrated in FIG. 4 may comprise a combination of network hub(s), network switch(es), and network router(s) connecting the devices 1a-1n/2a-2m or the sensing systems 20069 to the cloud-base system 20064. One or more of the devices 1a-1n/2a-2m or the sensing systems 20069 coupled to the network hub 20061 or network switch 20062 may collect data in real-time and transfer the data to cloud computers for data processing and manipulation. It will be appreciated that cloud computing relies on sharing computing resources rather than having local servers or personal devices to handle software applications. The word "cloud" may be used as a metaphor for "the Internet," although the term is not limited as such. Accordingly, the term "cloud computing" may be used herein to refer to "a type of Internet-based computing," where different services-such as servers, storage, and applications-are delivered to the modular communication hub 20065 and/or computer system 20063 located in the surgical theater (e.g., a fixed, mobile, temporary, or field operating room or space) and to devices connected to the modular communication hub 20065 and/or computer system 20063 through the Internet. The cloud infrastructure may be maintained by a cloud service provider. In this context, the cloud service provider may be the entity that coordinates the usage and control of the devices 1a-1n/2a-2m located in one or more operating theaters. The cloud computing services can perform a large number of calculations based on the data gathered by smart surgical instruments, robots, sensing systems, and other computerized devices located in the operating theater. The hub hardware enables multiple devices, sensing systems, and/or connections to be connected to a computer that communicates with the cloud computing resources and storage.

Applying cloud computer data processing techniques on the data collected by the devices 1a-1n/2a-2m, the surgical data network can provide improved surgical outcomes, reduced costs, and improved patient satisfaction. At least some of the devices 1a-1n/2a-2m may be employed to view tissue states to assess leaks or perfusion of sealed tissue after a tissue sealing and cutting procedure. At least some of the devices 1a-1n/2a-2m may be employed to identify pathology, such as the effects of diseases, using the cloud-based computing to examine data including images of samples of body tissue for diagnostic purposes. This may include localization and margin confirmation of tissue and phenotypes. At least some of the devices 1a-1n/2a-2m may be employed to identify anatomical structures of the body using a variety of sensors integrated with imaging devices and techniques such as overlaying images captured by multiple imaging devices. The data gathered by the devices 1a-1n/2a-2m, including image data, may be transferred to the cloud computing system 20064 or the local computer system 20063 or both for data processing and manipulation including image processing and manipulation. The data may be analyzed to improve surgical procedure outcomes by determining if further treatment, such as the application of endoscopic intervention, emerging technologies, a targeted radiation, targeted intervention, and precise robotics to tissue-specific sites and conditions, may be pursued. Such data analysis may further employ outcome analytics processing and using standardized approaches may provide beneficial feedback to either confirm surgical treatments and the behavior of the surgeon or suggest modifications to surgical treatments and the behavior of the surgeon.

Applying cloud computer data processing techniques on the measurement data collected by the sensing systems 20069, the surgical data network can provide improved surgical outcomes, improved recovery outcomes, reduced costs, and improved patient satisfaction. At least some of the sensing systems 20069 may be employed to assess physiological conditions of a surgeon operating on a patient or a patient being prepared for a surgical procedure or a patient recovering after a surgical procedure. The cloud-based computing system 20064 may be used to monitor biomarkers associated with a surgeon or a patient in real-time and to generate surgical plans based at least on measurement data gathered prior to a surgical procedure, provide control signals to the surgical instruments during a surgical procedure, and notify a patient of a complication during post-surgical period.

The operating theater devices 1a-1n may be connected to the modular communication hub 20065 over a wired channel or a wireless channel depending on the configuration of the devices 1a-1n to a network hub 20061. The network hub 20061 may be implemented, in one aspect, as a local network broadcast device that works on the physical layer of the Open System Interconnection (OSI) model. The network hub may provide connectivity to the devices 1a-1n located in the same operating theater network. The network hub 20061 may collect data in the form of packets and sends them to the router in half duplex mode. The network hub 20061 may not store any media access control/Internet Protocol (MAC/IP) to transfer the device data. Only one of the devices 1a-1n can send data at a time through the network hub 20061. The network hub 20061 may not have routing tables or intelligence regarding where to send information and broadcasts all network data across each connection and to a remote server 20067 of the cloud computing system 20064. The network hub 20061 can detect basic network errors such as collisions but having all information broadcast to multiple ports can be a security risk and cause bottlenecks.

The operating theater devices 2a-2m may be connected to a network switch 20062 over a wired channel or a wireless channel. The network switch 20062 works in the data link layer of the OSI model. The network switch 20062 may be a multicast device for connecting the devices 2a-2m located in the same operating theater to the network. The network switch 20062 may send data in the form of frames to the network router 20066 and may work in full duplex mode. Multiple devices 2a-2m can send data at the same time through the network switch 20062. The network switch 20062 stores and uses MAC addresses of the devices 2a-2m to transfer data.

The network hub 20061 and/or the network switch 20062 may be coupled to the network router 20066 for connection to the cloud computing system 20064. The network router 20066 works in the network layer of the OSI model. The network router 20066 creates a route for transmitting data packets received from the network hub 20061 and/or network switch 20062 to cloud-based computer resources for further processing and manipulation of the data collected by any one of or all the devices 1a-1n/2a-2m and wearable sensing system 20011. The network router 20066 may be employed to connect two or more different networks located in different locations, such as, for example, different operating theaters of the same healthcare facility or different networks located in different operating theaters of different healthcare facilities. The network router 20066 may send data in the form of packets to the cloud computing system 20064 and works in full duplex mode. Multiple devices can send data at the same time. The network router 20066 may use IP addresses to transfer data.

In an example, the network hub 20061 may be implemented as a USB hub, which allows multiple USB devices to be connected to a host computer. The USB hub may expand a single USB port into several tiers so that there are more ports available to connect devices to the host system computer. The network hub 20061 may include wired or wireless capabilities to receive information over a wired channel or a wireless channel. In one aspect, a wireless USB short-range, high-bandwidth wireless radio communication protocol may be employed for communication between the devices 1a-1n and devices 2a-2m located in the operating theater.

In examples, the operating theater devices 1a-1n/2a-2m and/or the sensing systems 20069 may communicate to the modular communication hub 20065 via Bluetooth wireless technology standard for exchanging data over short distances (using short-wavelength UHF radio waves in the ISM band from 2.4 to 2.485 GHz) from fixed and mobile devices and building personal area networks (PANs). The operating theater devices 1a-1n/2a-2m and/or the sensing systems 20069 may communicate to the modular communication hub 20065 via a number of wireless or wired communication standards or protocols, including but not limited to Bluetooth, Low-Energy Bluetooth, near-field communication (NFC), Wi-Fi (IEEE 802.11 family), WiMAX (IEEE 802.16 family), IEEE 802.20, new radio (NR), long-term evolution (LTE), and Ev-DO, HSPA+, HSDPA+, HSUPA+, EDGE, GSM, GPRS, CDMA, TDMA, DECT, and Ethernet derivatives thereof, as well as any other wireless and wired protocols that are designated as 3G, 4G, 5G, and beyond. The computing module may include a plurality of communication modules. For instance, a first communication module may be dedicated to shorter-range wireless communications such as Wi-Fi and Bluetooth Low-Energy Bluetooth, Bluetooth Smart, and a second communication module may be dedicated to longer-range wireless communications such as GPS, EDGE, GPRS, CDMA, WiMAX, LTE, Ev-DO, HSPA+, HSDPA+, HSUPA+, EDGE, GSM, GPRS, CDMA, TDMA, and others.

The modular communication hub 20065 may serve as a central connection for one or more of the operating theater devices 1a-1n/2a-2m and/or the sensing systems 20069 and may handle a data type known as frames. Frames may carry the data generated by the devices 1a-1n/2a-2m and/or the sensing systems 20069. When a frame is received by the modular communication hub 20065, it may be amplified and/or sent to the network router 20066, which may transfer the data to the cloud computing system 20064 or the local computer system 20063 by using a number of wireless or wired communication standards or protocols, as described herein.

The modular communication hub 20065 can be used as a standalone device or be connected to compatible network hubs 20061 and network switches 20062 to form a larger network. The modular communication hub 20065 can be generally easy to install, configure, and maintain, making it a good option for networking the operating theater devices 1a-1n/2a-2m.

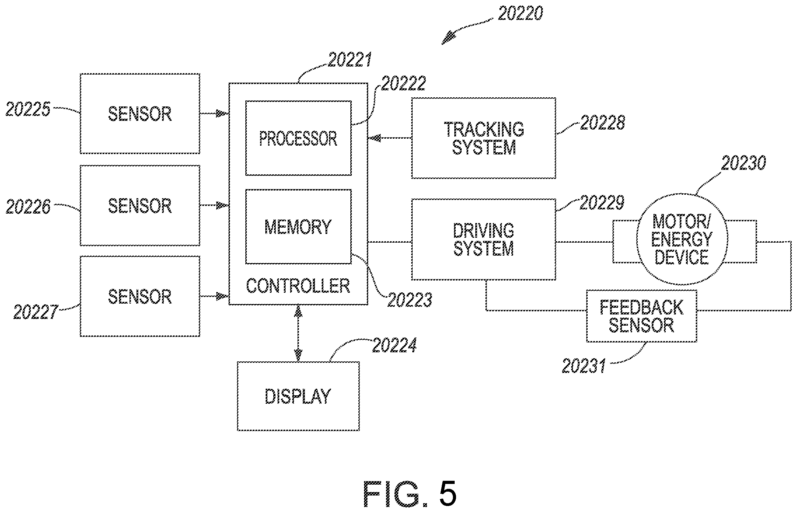

FIG. 5 illustrates a logical diagram of a control system 20220 of a surgical instrument or a surgical tool in accordance with one or more aspects of the present disclosure. The surgical instrument or the surgical tool may be configurable. The surgical instrument may include surgical fixtures specific to the procedure at-hand, such as imaging devices, surgical staplers, energy devices, endocutter devices, or the like. For example, the surgical instrument may include any of a powered stapler, a powered stapler generator, an energy device, an advanced energy device, an advanced energy jaw device, an endocutter clamp, an energy device generator, an in-operating-room imaging system, a smoke evacuator, a suction-irrigation device, an insufflation system, or the like. The system 20220 may comprise a control circuit. The control circuit may include a microcontroller 20221 comprising a processor 20222 and a memory 20223. One or more of sensors 20225, 20226, 20227, for example, provide real-time feedback to the processor 20222. A motor 20230, driven by a motor driver 20229, operably couples a longitudinally movable displacement member to drive the I-beam knife element. A tracking system 20228 may be configured to determine the position of the longitudinally movable displacement member. The position information may be provided to the processor 20222, which can be programmed or configured to determine the position of the longitudinally movable drive member as well as the position of a firing member, firing bar, and I-beam knife element. Additional motors may be provided at the tool driver interface to control I-beam firing, closure tube travel, shaft rotation, and articulation. A display 20224 may display a variety of operating conditions of the instruments and may include touch screen functionality for data input. Information displayed on the display 20224 may be overlaid with images acquired via endoscopic imaging modules.

The microcontroller 20221 may be any single-core or multicore processor such as those known under the trade name ARM Cortex by Texas Instruments. In one aspect, the main microcontroller 20221 may be an LM4F230H5QR ARM Cortex-M4F Processor Core, available from Texas Instruments, for example, comprising an on-chip memory of 256 KB single-cycle flash memory, or other non-volatile memory, up to 40 MHz, a prefetch buffer to improve performance above 40 MHz, a 32 KB single-cycle SRAM, and internal ROM loaded with StellarisWare^{®} software, a 2 KB EEPROM, one or more PWM modules, one or more QEI analogs, and/or one or more 12-bit ADCs with 12 analog input channels, details of which are available for the product datasheet.

The microcontroller 20221 may comprise a safety controller comprising two controller-based families such as TMS570 and RM4x, known under the trade name Hercules ARM Cortex R4, also by Texas Instruments. The safety controller may be configured specifically for IEC 61508 and ISO 26262 safety critical applications, among others, to provide advanced integrated safety features while delivering scalable performance, connectivity, and memory options.

The microcontroller 20221 may be programmed to perform various functions such as precise control over the speed and position of the knife and articulation systems. In one aspect, the microcontroller 20221 may include a processor 20222 and a memory 20223. The electric motor 20230 may be a brushed direct current (DC) motor with a gearbox and mechanical links to an articulation or knife system. In one aspect, a motor driver 20229 may be an A3941 available from Allegro Microsystems, Inc. Other motor drivers may be readily substituted for use in the tracking system 20228 comprising an absolute positioning system. A detailed description of an absolute positioning system is described in U.S. Patent Application Publication No. 2017/0296213, titled SYSTEMS AND METHODS FOR CONTROLLING A SURGICAL STAPLING AND CUTTING INSTRUMENT, which published on October 19, 2017.

The microcontroller 20221 may be programmed to provide precise control over the speed and position of displacement members and articulation systems. The microcontroller 20221 may be configured to compute a response in the software of the microcontroller 20221. The computed response may be compared to a measured response of the actual system to obtain an "observed" response, which is used for actual feedback decisions. The observed response may be a favorable, tuned value that balances the smooth, continuous nature of the simulated response with the measured response, which can detect outside influences on the system.

The motor 20230 may be controlled by the motor driver 20229 and can be employed by the firing system of the surgical instrument or tool. In various forms, the motor 20230 may be a brushed DC driving motor having a maximum rotational speed of approximately 25,000 RPM. In some examples, the motor 20230 may include a brushless motor, a cordless motor, a synchronous motor, a stepper motor, or any other suitable electric motor. The motor driver 20229 may comprise an H-bridge driver comprising field-effect transistors (FETs), for example. The motor 20230 can be powered by a power assembly releasably mounted to the handle assembly or tool housing for supplying control power to the surgical instrument or tool. The power assembly may comprise a battery which may include a number of battery cells connected in series that can be used as the power source to power the surgical instrument or tool. In certain circumstances, the battery cells of the power assembly may be replaceable and/or rechargeable. In at least one example, the battery cells can be lithium-ion batteries which can be couplable to and separable from the power assembly.

The motor driver 20229 may be an A3941 available from Allegro Microsystems, Inc. A3941 may be a full-bridge controller for use with external N-channel power metal-oxide semiconductor field-effect transistors (MOSFETs) specifically designed for inductive loads, such as brush DC motors. The driver 20229 may comprise a unique charge pump regulator that can provide full (>10 V) gate drive for battery voltages down to 7 V and can allow the A3941 to operate with a reduced gate drive, down to 5.5 V. A bootstrap capacitor may be employed to provide the above battery supply voltage required for N-channel MOSFETs. An internal charge pump for the high-side drive may allow DC (100% duty cycle) operation. The full bridge can be driven in fast or slow decay modes using diode or synchronous rectification. In the slow decay mode, current recirculation can be through the high-side or the low-side FETs. The power FETs may be protected from shoot-through by resistor-adjustable dead time. Integrated diagnostics provide indications of undervoltage, overtemperature, and power bridge faults and can be configured to protect the power MOSFETs under most short circuit conditions. Other motor drivers may be readily substituted for use in the tracking system 20228 comprising an absolute positioning system.

The tracking system 20228 may comprise a controlled motor drive circuit arrangement comprising a position sensor 20225 according to one aspect of this disclosure. The position sensor 20225 for an absolute positioning system may provide a unique position signal corresponding to the location of a displacement member. In some examples, the displacement member may represent a longitudinally movable drive member comprising a rack of drive teeth for meshing engagement with a corresponding drive gear of a gear reducer assembly. In some examples, the displacement member may represent the firing member, which could be adapted and configured to include a rack of drive teeth. In some examples, the displacement member may represent a firing bar or the I-beam, each of which can be adapted and configured to include a rack of drive teeth. Accordingly, as used herein, the term displacement member can be used generically to refer to any movable member of the surgical instrument or tool such as the drive member, the firing member, the firing bar, the I-beam, or any element that can be displaced. In one aspect, the longitudinally movable drive member can be coupled to the firing member, the firing bar, and the I-beam. Accordingly, the absolute positioning system can, in effect, track the linear displacement of the I-beam by tracking the linear displacement of the longitudinally movable drive member. In various aspects, the displacement member may be coupled to any position sensor 20225 suitable for measuring linear displacement. Thus, the longitudinally movable drive member, the firing member, the firing bar, or the I-beam, or combinations thereof, may be coupled to any suitable linear displacement sensor. Linear displacement sensors may include contact or non-contact displacement sensors. Linear displacement sensors may comprise linear variable differential transformers (LVDT), differential variable reluctance transducers (DVRT), a slide potentiometer, a magnetic sensing system comprising a movable magnet and a series of linearly arranged Hall effect sensors, a magnetic sensing system comprising a fixed magnet and a series of movable, linearly arranged Hall effect sensors, an optical sensing system comprising a movable light source and a series of linearly arranged photo diodes or photo detectors, an optical sensing system comprising a fixed light source and a series of movable linearly, arranged photodiodes or photodetectors, or any combination thereof.

The electric motor 20230 can include a rotatable shaft that operably interfaces with a gear assembly that is mounted in meshing engagement with a set, or rack, of drive teeth on the displacement member. A sensor element may be operably coupled to a gear assembly such that a single revolution of the position sensor 20225 element corresponds to some linear longitudinal translation of the displacement member. An arrangement of gearing and sensors can be connected to the linear actuator, via a rack and pinion arrangement, or a rotary actuator, via a spur gear or other connection. A power source may supply power to the absolute positioning system and an output indicator may display the output of the absolute positioning system. The displacement member may represent the longitudinally movable drive member comprising a rack of drive teeth formed thereon for meshing engagement with a corresponding drive gear of the gear reducer assembly. The displacement member may represent the longitudinally movable firing member, firing bar, I-beam, or combinations thereof.

A single revolution of the sensor element associated with the position sensor 20225 may be equivalent to a longitudinal linear displacement d1 of the displacement member, where d1 is the longitudinal linear distance that the displacement member moves from point "a" to point "b" after a single revolution of the sensor element coupled to the displacement member. The sensor arrangement may be connected via a gear reduction that results in the position sensor 20225 completing one or more revolutions for the full stroke of the displacement member. The position sensor 20225 may complete multiple revolutions for the full stroke of the displacement member.

A series of switches, where n is an integer greater than one, may be employed alone or in combination with a gear reduction to provide a unique position signal for more than one revolution of the position sensor 20225. The state of the switches may be fed back to the microcontroller 20221 that applies logic to determine a unique position signal corresponding to the longitudinal linear displacement d1 + d2 + ... dn of the displacement member. The output of the position sensor 20225 is provided to the microcontroller 20221. The position sensor 20225 of the sensor arrangement may comprise a magnetic sensor, an analog rotary sensor like a potentiometer, or an array of analog Hall-effect elements, which output a unique combination of position signals or values.

The position sensor 20225 may comprise any number of magnetic sensing elements, such as, for example, magnetic sensors classified according to whether they measure the total magnetic field or the vector components of the magnetic field. The techniques used to produce both types of magnetic sensors may encompass many aspects of physics and electronics. The technologies used for magnetic field sensing may include search coil, fluxgate, optically pumped, nuclear precession, SQUID, Hall-effect, anisotropic magnetoresistance, giant magnetoresistance, magnetic tunnel junctions, giant magnetoimpedance, magnetostrictive/piezoelectric composites, magnetodiode, magnetotransistor, fiber-optic, magneto-optic, and microelectromechanical systems-based magnetic sensors, among others.

The position sensor 20225 for the tracking system 20228 comprising an absolute positioning system may comprise a magnetic rotary absolute positioning system. The position sensor 20225 may be implemented as an AS5055EQFT single-chip magnetic rotary position sensor available from Austria Microsystems, AG. The position sensor 20225 is interfaced with the microcontroller 20221 to provide an absolute positioning system. The position sensor 20225 may be a low-voltage and low-power component and may include four Hall-effect elements in an area of the position sensor 20225 that may be located above a magnet. A high-resolution ADC and a smart power management controller may also be provided on the chip. A coordinate rotation digital computer (CORDIC) processor, also known as the digit-by-digit method and Volder's algorithm, may be provided to implement a simple and efficient algorithm to calculate hyperbolic and trigonometric functions that require only addition, subtraction, bit-shift, and table lookup operations. The angle position, alarm bits, and magnetic field information may be transmitted over a standard serial communication interface, such as a serial peripheral interface (SPI) interface, to the microcontroller 20221. The position sensor 20225 may provide 12 or 14 bits of resolution. The position sensor 20225 may be an AS5055 chip provided in a small QFN 16-pin 4x4x0.85mm package.

The tracking system 20228 comprising an absolute positioning system may comprise and/or be programmed to implement a feedback controller, such as a PID, state feedback, and adaptive controller. A power source converts the signal from the feedback controller into a physical input to the system: in this case the voltage. Other examples include a PWM of the voltage, current, and force. Other sensor(s) may be provided to measure physical parameters of the physical system in addition to the position measured by the position sensor 20225. In some aspects, the other sensor(s) can include sensor arrangements such as those described in U.S. Patent No. 9,345,481, titled STAPLE CARTRIDGE TISSUE THICKNESS SENSOR SYSTEM, which issued on May 24, 2016; U.S. Patent Application Publication No. 2014/0263552, titled STAPLE CARTRIDGE TISSUE THICKNESS SENSOR SYSTEM, which published on September 18, 2014; and U.S. Patent Application Serial No. 15/628,175, titled TECHNIQUES FOR ADAPTIVE CONTROL OF MOTOR VELOCITY OF A SURGICAL STAPLING AND CUTTING INSTRUMENT, filed June 20, 2017. In a digital signal processing system, an absolute positioning system is coupled to a digital data acquisition system where the output of the absolute positioning system will have a finite resolution and sampling frequency. The absolute positioning system may comprise a compare-and-combine circuit to combine a computed response with a measured response using algorithms, such as a weighted average and a theoretical control loop, that drive the computed response towards the measured response. The computed response of the physical system may take into account properties like mass, inertia, viscous friction, inductance resistance, etc., to predict what the states and outputs of the physical system will be by knowing the input.

The absolute positioning system may provide an absolute position of the displacement member upon power-up of the instrument, without retracting or advancing the displacement member to a reset (zero or home) position as may be required with conventional rotary encoders that merely count the number of steps forwards or backwards that the motor 20230 has taken to infer the position of a device actuator, drive bar, knife, or the like.

A sensor 20226, such as, for example, a strain gauge or a micro-strain gauge, may be configured to measure one or more parameters of the end effector, such as, for example, the amplitude of the strain exerted on the anvil during a clamping operation, which can be indicative of the closure forces applied to the anvil. The measured strain may be converted to a digital signal and provided to the processor 20222. Alternatively, or in addition to the sensor 20226, a sensor 20227, such as, for example, a load sensor, can measure the closure force applied by the closure drive system to the anvil. The sensor 20227, such as, for example, a load sensor, can measure the firing force applied to an I-beam in a firing stroke of the surgical instrument or tool. The I-beam is configured to engage a wedge sled, which is configured to upwardly cam staple drivers to force out staples into deforming contact with an anvil. The I-beam also may include a sharpened cutting edge that can be used to sever tissue as the I-beam is advanced distally by the firing bar. Alternatively, a current sensor 20231 can be employed to measure the current drawn by the motor 20230. The force required to advance the firing member can correspond to the current drawn by the motor 20230, for example. The measured force may be converted to a digital signal and provided to the processor 20222.

For example, the strain gauge sensor 20226 can be used to measure the force applied to the tissue by the end effector. A strain gauge can be coupled to the end effector to measure the force on the tissue being treated by the end effector. A system for measuring forces applied to the tissue grasped by the end effector may comprise a strain gauge sensor 20226, such as, for example, a micro-strain gauge, that can be configured to measure one or more parameters of the end effector, for example. In one aspect, the strain gauge sensor 20226 can measure the amplitude or magnitude of the strain exerted on a jaw member of an end effector during a clamping operation, which can be indicative of the tissue compression. The measured strain can be converted to a digital signal and provided to a processor 20222 of the microcontroller 20221. A load sensor 20227 can measure the force used to operate the knife element, for example, to cut the tissue captured between the anvil and the staple cartridge. A magnetic field sensor can be employed to measure the thickness of the captured tissue. The measurement of the magnetic field sensor also may be converted to a digital signal and provided to the processor 20222.

The measurements of the tissue compression, the tissue thickness, and/or the force required to close the end effector on the tissue, as respectively measured by the sensors 20226, 20227, can be used by the microcontroller 20221 to characterize the selected position of the firing member and/or the corresponding value of the speed of the firing member. In one instance, a memory 20223 may store a technique, an equation, and/or a lookup table which can be employed by the microcontroller 20221 in the assessment.

The control system 20220 of the surgical instrument or tool also may comprise wired or wireless communication circuits to communicate with the surgical hub 20065 as shown in FIG. 4.

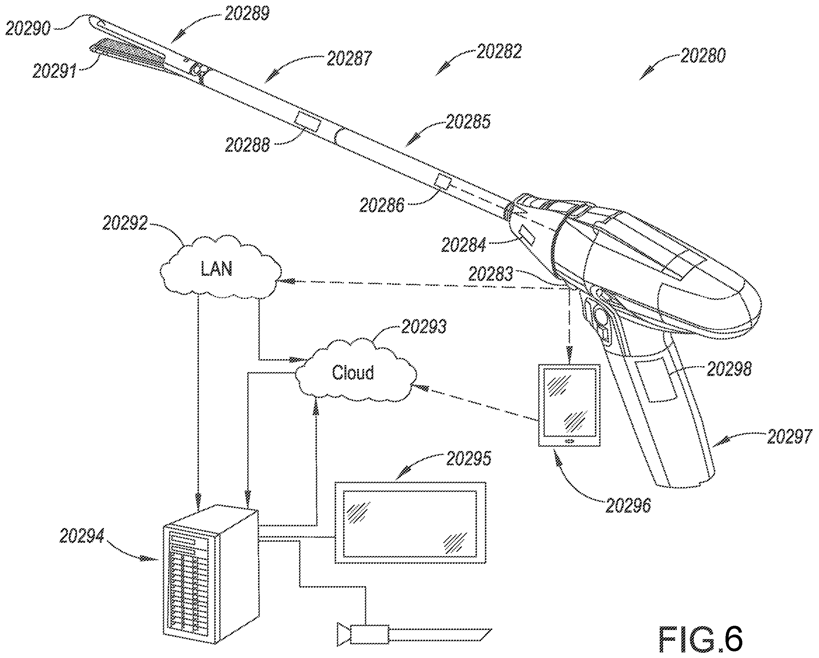

FIG. 6 illustrates an example surgical system 20280 in accordance with the present disclosure and may include a surgical instrument 20282 that can be in communication with a console 20294 or a portable device 20296 through a local area network 20292 and/or a cloud network 20293 via a wired and/or wireless connection. The console 20294 and the portable device 20296 may be any suitable computing device. The surgical instrument 20282 may include a handle 20297, an adapter 20285, and a loading unit 20287. The adapter 20285 releasably couples to the handle 20297 and the loading unit 20287 releasably couples to the adapter 20285 such that the adapter 20285 transmits a force from a drive shaft to the loading unit 20287. The adapter 20285 or the loading unit 20287 may include a force gauge (not explicitly shown) disposed therein to measure a force exerted on the loading unit 20287. The loading unit 20287 may include an end effector 20289 having a first jaw 20291 and a second jaw 20290. The loading unit 20287 may be an in-situ loaded or multi-firing loading unit (MFLU) that allows a clinician to fire a plurality of fasteners multiple times without requiring the loading unit 20287 to be removed from a surgical site to reload the loading unit 20287.

The first and second jaws 20291, 20290 may be configured to clamp tissue therebetween, fire fasteners through the clamped tissue, and sever the clamped tissue. The first jaw 20291 may be configured to fire at least one fastener a plurality of times or may be configured to include a replaceable multi-fire fastener cartridge including a plurality of fasteners (e.g., staples, clips, etc.) that may be fired more than one time prior to being replaced. The second jaw 20290 may include an anvil that deforms or otherwise secures the fasteners, as the fasteners are ejected from the multi-fire fastener cartridge.

The handle 20297 may include a motor that is coupled to the drive shaft to affect rotation of the drive shaft. The handle 20297 may include a control interface to selectively activate the motor. The control interface may include buttons, switches, levers, sliders, touchscreens, and any other suitable input mechanisms or user interfaces, which can be engaged by a clinician to activate the motor.

The control interface of the handle 20297 may be in communication with a controller 20298 of the handle 20297 to selectively activate the motor to affect rotation of the drive shafts. The controller 20298 may be disposed within the handle 20297 and may be configured to receive input from the control interface and adapter data from the adapter 20285 or loading unit data from the loading unit 20287. The controller 20298 may analyze the input from the control interface and the data received from the adapter 20285 and/or loading unit 20287 to selectively activate the motor. The handle 20297 may also include a display that is viewable by a clinician during use of the handle 20297. The display may be configured to display portions of the adapter or loading unit data before, during, or after firing of the instrument 20282.

The adapter 20285 may include an adapter identification device 20284 disposed therein and the loading unit 20287 may include a loading unit identification device 20288 disposed therein. The adapter identification device 20284 may be in communication with the controller 20298, and the loading unit identification device 20288 may be in communication with the controller 20298. It will be appreciated that the loading unit identification device 20288 may be in communication with the adapter identification device 20284, which relays or passes communication from the loading unit identification device 20288 to the controller 20298.

The adapter 20285 may also include a plurality of sensors 20286 (one shown) disposed thereabout to detect various conditions of the adapter 20285 or of the environment (e.g., if the adapter 20285 is connected to a loading unit, if the adapter 20285 is connected to a handle, if the drive shafts are rotating, the torque of the drive shafts, the strain of the drive shafts, the temperature within the adapter 20285, a number of firings of the adapter 20285, a peak force of the adapter 20285 during firing, a total amount of force applied to the adapter 20285, a peak retraction force of the adapter 20285, a number of pauses of the adapter 20285 during firing, etc.). The plurality of sensors 20286 may provide an input to the adapter identification device 20284 in the form of data signals. The data signals of the plurality of sensors 20286 may be stored within or be used to update the adapter data stored within the adapter identification device 20284. The data signals of the plurality of sensors 20286 may be analog or digital. The plurality of sensors 20286 may include a force gauge to measure a force exerted on the loading unit 20287 during firing.

The handle 20297 and the adapter 20285 can be configured to interconnect the adapter identification device 20284 and the loading unit identification device 20288 with the controller 20298 via an electrical interface. The electrical interface may be a direct electrical interface (i.e., include electrical contacts that engage one another to transmit energy and signals therebetween). Additionally, or alternatively, the electrical interface may be a non-contact electrical interface to wirelessly transmit energy and signals therebetween (e.g., inductively transfer). It is also contemplated that the adapter identification device 20284 and the controller 20298 may be in wireless communication with one another via a wireless connection separate from the electrical interface.

The handle 20297 may include a transceiver 20283 that is configured to transmit instrument data from the controller 20298 to other components of the system 20280 (e.g., the LAN 20292, the cloud 20293, the console 20294, or the portable device 20296). The controller 20298 may also transmit instrument data and/or measurement data associated with one or more sensors 20286 to a surgical hub. The transceiver 20283 may receive data (e.g., cartridge data, loading unit data, adapter data, or other notifications) from the surgical hub 20270. The transceiver 20283 may receive data (e.g., cartridge data, loading unit data, or adapter data) from the other components of the system 20280. For example, the controller 20298 may transmit instrument data including a serial number of an attached adapter (e.g., adapter 20285) attached to the handle 20297, a serial number of a loading unit (e.g., loading unit 20287) attached to the adapter 20285, and a serial number of a multi-fire fastener cartridge loaded into the loading unit to the console 20294. Thereafter, the console 20294 may transmit data (e.g., cartridge data, loading unit data, or adapter data) associated with the attached cartridge, loading unit, and adapter, respectively, back to the controller 20298. The controller 20298 can display messages on the local instrument display or transmit the message, via transceiver 20283, to the console 20294 or the portable device 20296 to display the message on the display 20295 or portable device screen, respectively.

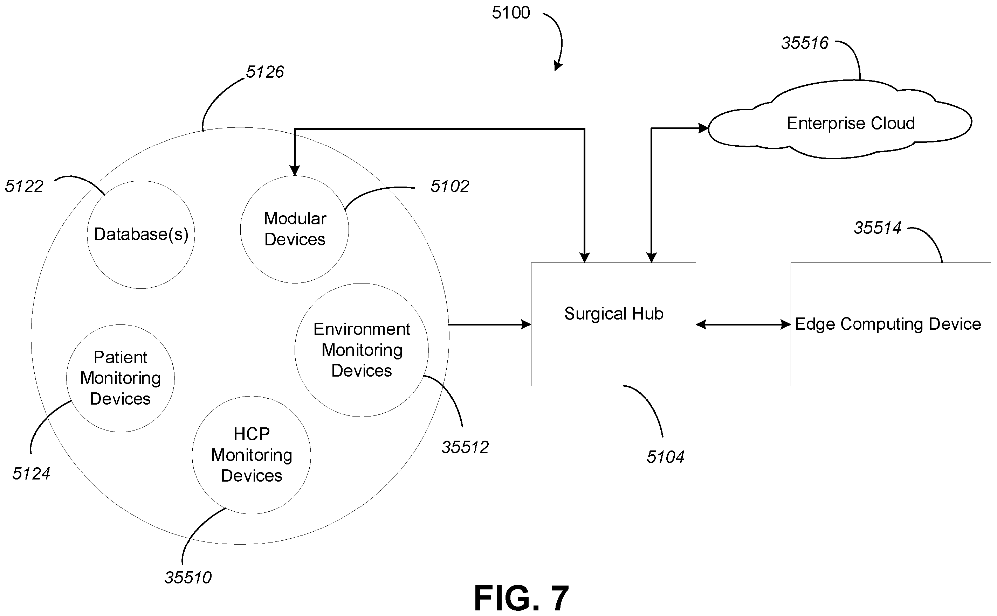

FIG. 7 illustrates a diagram of a situationally aware surgical system 5100, in accordance with at least one aspect of the present disclosure. The data sources 5126 may include, for example, the modular devices 5102 (which can include sensors configured to detect parameters associated with the patient, HCPs and environment and/or the modular device itself), databases 5122 (e.g., an EMR database containing patient records), patient monitoring devices 5124 (e.g., a blood pressure (BP) monitor and an electrocardiography (EKG) monitor), HCP monitoring devices 35510, and/or environment monitoring devices 35512. The surgical hub 5104 can be configured to derive the contextual information pertaining to the surgical procedure from the data based upon, for example, the particular combination(s) of received data or the particular order in which the data is received from the data sources 5126. The contextual information inferred from the received data can include, for example, the type of surgical procedure being performed, the particular step of the surgical procedure that the surgeon is performing, the type of tissue being operated on, or the body cavity that is the subject of the procedure. This ability by some aspects of the surgical hub 5104 to derive or infer information related to the surgical procedure from received data can be referred to as "situational awareness." For example, the surgical hub 5104 can incorporate a situational awareness system, which is the hardware and/or programming associated with the surgical hub 5104 that derives contextual information pertaining to the surgical procedure from the received data and/or a surgical plan information received from the edge computing system 35514 or an enterprise cloud server 35516.

The situational awareness system of the surgical hub 5104 can be configured to derive the contextual information from the data received from the data sources 5126 in a variety of different ways. For example, the situational awareness system can include a pattern recognition system, or machine learning system (e.g., an artificial neural network), that has been trained on training data to correlate various inputs (e.g., data from database(s) 5122, patient monitoring devices 5124, modular devices 5102, HCP monitoring devices 35510, and/or environment monitoring devices 35512) to corresponding contextual information regarding a surgical procedure. A machine learning system can be trained to accurately derive contextual information regarding a surgical procedure from the provided inputs. In examples, the situational awareness system can include a lookup table storing pre-characterized contextual information regarding a surgical procedure in association with one or more inputs (or ranges of inputs) corresponding to the contextual information. In response to a query with one or more inputs, the lookup table can return the corresponding contextual information for the situational awareness system for controlling the modular devices 5102. In examples, the contextual information received by the situational awareness system of the surgical hub 5104 can be associated with a particular control adjustment or set of control adjustments for one or more modular devices 5102. In examples, the situational awareness system can include a further machine learning system, lookup table, or other such system, which generates or retrieves one or more control adjustments for one or more modular devices 5102 when provided the contextual information as input.

A surgical hub 5104 incorporating a situational awareness system can provide a number of benefits for the surgical system 5100. One benefit may include improving the interpretation of sensed and collected data, which would in turn improve the processing accuracy and/or the usage of the data during the course of a surgical procedure. To return to a previous example, a situationally aware surgical hub 5104 could determine what type of tissue was being operated on; therefore, when an unexpectedly high force to close the surgical instrument's end effector is detected, the situationally aware surgical hub 5104 could correctly ramp up or ramp down the motor of the surgical instrument for the type of tissue.

The type of tissue being operated can affect the adjustments that are made to the compression rate and load thresholds of a surgical stapling and cutting instrument for a particular tissue gap measurement. A situationally aware surgical hub 5104 could infer whether a surgical procedure being performed is a thoracic or an abdominal procedure, allowing the surgical hub 5104 to determine whether the tissue clamped by an end effector of the surgical stapling and cutting instrument is lung (for a thoracic procedure) or stomach (for an abdominal procedure) tissue. The surgical hub 5104 could then adjust the compression rate and load thresholds of the surgical stapling and cutting instrument appropriately for the type of tissue.

The type of body cavity being operated in during an insufflation procedure can affect the function of a smoke evacuator. A situationally aware surgical hub 5104 could determine whether the surgical site is under pressure (by determining that the surgical procedure is utilizing insufflation) and determine the procedure type. As a procedure type can be generally performed in a specific body cavity, the surgical hub 5104 could then control the motor rate of the smoke evacuator appropriately for the body cavity being operated in. Thus, a situationally aware surgical hub 5104 could provide a consistent amount of smoke evacuation for both thoracic and abdominal procedures.

The type of procedure being performed can affect the optimal energy level for an ultrasonic surgical instrument or radio frequency (RF) electrosurgical instrument to operate at. Arthroscopic procedures, for example, may require higher energy levels because the end effector of the ultrasonic surgical instrument or RF electrosurgical instrument is immersed in fluid. A situationally aware surgical hub 5104 could determine whether the surgical procedure is an arthroscopic procedure. The surgical hub 5104 could then adjust the RF power level or the ultrasonic amplitude of the generator (e.g., "energy level") to compensate for the fluid filled environment. Relatedly, the type of tissue being operated on can affect the optimal energy level for an ultrasonic surgical instrument or RF electrosurgical instrument to operate at. A situationally aware surgical hub 5104 could determine what type of surgical procedure is being performed and then customize the energy level for the ultrasonic surgical instrument or RF electrosurgical instrument, respectively, according to the expected tissue profile for the surgical procedure. Furthermore, a situationally aware surgical hub 5104 can be configured to adjust the energy level for the ultrasonic surgical instrument or RF electrosurgical instrument throughout the course of a surgical procedure, rather than just on a procedure-by-procedure basis. A situationally aware surgical hub 5104 could determine what step of the surgical procedure is being performed or will subsequently be performed and then update the control algorithms for the generator and/or ultrasonic surgical instrument or RF electrosurgical instrument to set the energy level at a value appropriate for the expected tissue type according to the surgical procedure step.

In examples, data can be drawn from additional data sources 5126 to improve the conclusions that the surgical hub 5104 draws from one data source 5126. A situationally aware surgical hub 5104 could augment data that it receives from the modular devices 5102 with contextual information that it has built up regarding the surgical procedure from other data sources 5126. For example, a situationally aware surgical hub 5104 can be configured to determine whether hemostasis has occurred (e.g., whether bleeding at a surgical site has stopped) according to video or image data received from a medical imaging device. The surgical hub 5104 can be further configured to compare a physiologic measurement (e.g., blood pressure sensed by a BP monitor communicably connected to the surgical hub 5104) with the visual or image data of hemostasis (e.g., from a medical imaging device communicably coupled to the surgical hub 5104) to make a determination on the integrity of the staple line or tissue weld. The situational awareness system of the surgical hub 5104 can consider the physiological measurement data to provide additional context in analyzing the visualization data. The additional context can be useful when the visualization data may be inconclusive or incomplete on its own.

For example, a situationally aware surgical hub 5104 could proactively activate the generator to which an RF electrosurgical instrument is connected if it determines that a subsequent step of the procedure requires the use of the instrument. Proactively activating the energy source can allow the instrument to be ready for use as soon as the preceding step of the procedure is completed.

The situationally aware surgical hub 5104 could determine whether the current or subsequent step of the surgical procedure requires a different view or degree of magnification on the display according to the feature(s) at the surgical site that the surgeon is expected to need to view. The surgical hub 5104 could proactively change the displayed view (supplied by, e.g., a medical imaging device for the visualization system) accordingly so that the display automatically adjusts throughout the surgical procedure.

The situationally aware surgical hub 5104 could determine which step of the surgical procedure is being performed or will subsequently be performed and whether particular data or comparisons between data will be required for that step of the surgical procedure. The surgical hub 5104 can be configured to automatically call up data screens based upon the step of the surgical procedure being performed, without waiting for the surgeon to ask for the particular information.

Errors may be checked during the setup of the surgical procedure or during the course of the surgical procedure. For example, the situationally aware surgical hub 5104 could determine whether the operating theater is setup properly or optimally for the surgical procedure to be performed. The surgical hub 5104 can be configured to determine the type of surgical procedure being performed, retrieve the corresponding checklists, product location, or setup needs (e.g., from a memory), and then compare the current operating theater layout to the standard layout for the type of surgical procedure that the surgical hub 5104 determines is being performed. In some exemplifications, the surgical hub 5104 can compare the list of items for the procedure and/or a list of devices paired with the surgical hub 5104 to a recommended or anticipated manifest of items and/or devices for the given surgical procedure. If there are any discontinuities between the lists, the surgical hub 5104 can provide an alert indicating that a particular modular device 5102, patient monitoring device 5124, HCP monitoring devices 35510, environment monitoring devices 35512, and/or other surgical item is missing. In some examples, the surgical hub 5104 can determine the relative distance or position of the modular devices 5102 and patient monitoring devices 5124 via proximity sensors, for example. The surgical hub 5104 can compare the relative positions of the devices to a recommended or anticipated layout for the particular surgical procedure. If there are any discontinuities between the layouts, the surgical hub 5104 can be configured to provide an alert indicating that the current layout for the surgical procedure deviates from the recommended layout.

The situationally aware surgical hub 5104 could determine whether the surgeon (or other HCP(s)) was making an error or otherwise deviating from the expected course of action during the course of a surgical procedure. For example, the surgical hub 5104 can be configured to determine the type of surgical procedure being performed, retrieve the corresponding list of steps or order of equipment usage (e.g., from a memory), and then compare the steps being performed or the equipment being used during the course of the surgical procedure to the expected steps or equipment for the type of surgical procedure that the surgical hub 5104 determined is being performed. The surgical hub 5104 can provide an alert indicating that an unexpected action is being performed or an unexpected device is being utilized at the particular step in the surgical procedure.

The surgical instruments (and other modular devices 5102) may be adjusted for the particular context of each surgical procedure (such as adjusting to different tissue types) and validating actions during a surgical procedure. Next steps, data, and display adjustments may be provided to surgical instruments (and other modular devices 5102) in the surgical theater according to the specific context of the procedure.

Surgical autonomous systems, devices, and methods may include aspects of integration with other medical equipment, data sources, processes, and institutions. Surgical autonomous systems, devices, and methods may include aspects of integration with a computer-implemented interactive surgical system and/or with one or more elements of a computer-implemented interactive surgical system, for example. Surgical system, surgical autonomous system, and autonomous surgical system may be interchangeably as described herein.

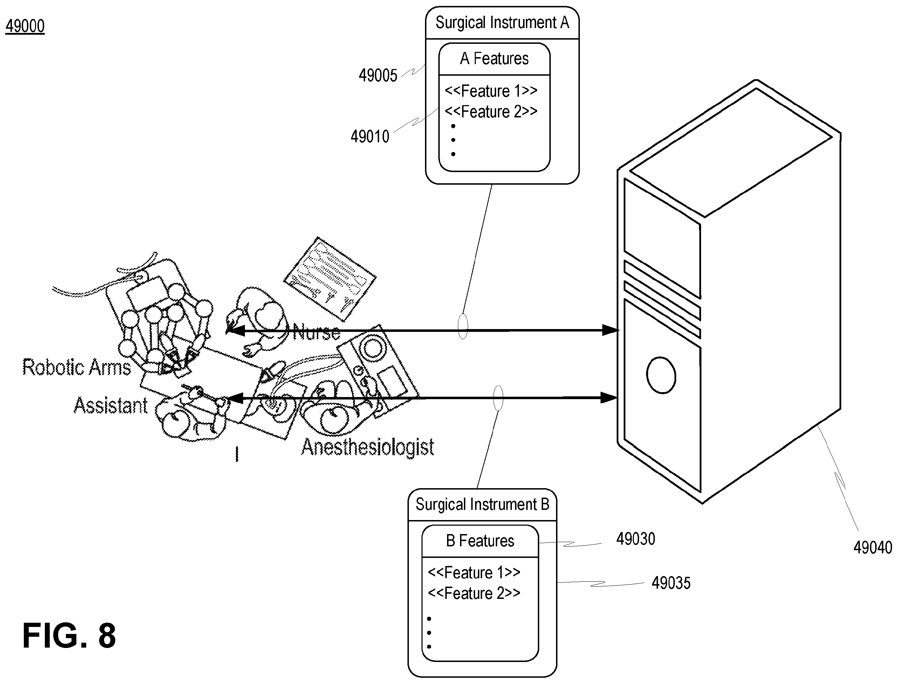

Referring to FIG. 8, an overview of the surgical autonomous system 49000 may be provided. Surgical instrument A 49005 and/or surgical instrument B 49035 may be used in a surgical procedure as part of the surgical system 49000. The surgical hub 4900 may be configured to coordinate information flow to a display of the surgical instrument. For example, the surgical hub may be described in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018. Example surgical instruments that are suitable for use with the surgical system 49000 are described under the heading "Surgical Instrument Hardware" and in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, for example.

FIG. 8 shows an example of a surgical autonomous system 49000. The system 49000 may be used to perform a surgical procedure on a patient who is lying down on an operating table in a surgical operating room. A robotic system may be used in the surgical procedure as a part of the surgical system. For example, the robotic system may be described in U.S.

Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018. The robotic hub may be used to process the images of the surgical site for subsequent display to the surgeon through the surgeon's console.

Other types of robotic systems may be readily adapted for use with the surgical system 49000. Various examples of robotic systems and surgical tools that are suitable for use with the present disclosure are described in U.S. Patent Application Publication No. US 2019-0201137 A1 (U.S. Patent Application No. 16/209,407), titled METHOD OF ROBOTIC HUB COMMUNICATION, DETECTION, AND CONTROL, filed December 4, 2018.

Various examples of cloud-based analytics that are performed by the cloud, and are suitable for use with the present disclosure, are described in U.S. Patent Application Publication No. US 2019-0206569 A1 (U.S. Patent Application No. 16/209,403), titled METHOD OF CLOUD BASED DATA ANALYTICS FOR USE WITH THE HUB, filed December 4, 2018.

In various aspects, an imaging device may be used in the surgical system and may include at least one image sensor and one or more optical components. Suitable image sensors may include, but are not limited to, Charge-Coupled Device (CCD) sensors and Complementary Metal-Oxide Semiconductor (CMOS) sensors.

The optical components of the imaging device may include one or more illumination sources and/or one or more lenses. The one or more illumination sources may be directed to illuminate portions of the surgical field. The one or more image sensors may receive light reflected or refracted from the surgical field, including light reflected or refracted from tissue and/or surgical instruments.

The one or more illumination sources may be configured to radiate electromagnetic energy in the visible spectrum as well as the invisible spectrum. The visible spectrum, sometimes referred to as the optical spectrum or luminous spectrum, is that portion of the electromagnetic spectrum that is visible to (e.g., can be detected by) the human eye and may be referred to as visible light or simply light. A typical human eye will respond to wavelengths in air that are from about 380 nm to about 750 nm.

The invisible spectrum (e.g., the non-luminous spectrum) is that portion of the electromagnetic spectrum that lies below and above the visible spectrum (i.e., wavelengths below about 380 nm and above about 750 nm). The invisible spectrum is not detectable by the human eye. Wavelengths greater than about 750 nm are longer than the red visible spectrum, and they become invisible infrared (IR), microwave, and radio electromagnetic radiation. Wavelengths less than about 380 nm are shorter than the violet spectrum, and they become invisible ultraviolet, x-ray, and gamma ray electromagnetic radiation.

In various aspects, the imaging device may be configured for use in a minimally invasive procedure. Examples of imaging devices suitable for use with the present disclosure include, but not limited to, an arthroscope, angioscope, bronchoscope, choledochoscope, colonoscope, cytoscope, duodenoscope, enteroscope, esophagogastro-duodenoscope (gastroscope), endoscope, laryngoscope, nasopharyngo-neproscope, sigmoidoscope, thoracoscope, and ureteroscope.

The imaging device may employ multi-spectrum monitoring to discriminate topography and underlying structures. A multi-spectral image is one that captures image data within specific wavelength ranges across the electromagnetic spectrum. The wavelengths may be separated by filters or by the use of instruments that are sensitive to particular wavelengths, including light from frequencies beyond the visible light range, e.g., IR and ultraviolet. Spectral imaging can allow extraction of additional information the human eye fails to capture with its receptors for red, green, and blue. The use of multi-spectral imaging is described in greater detail under the heading "Advanced Imaging Acquisition Module" in .S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018. Multi-spectrum monitoring can be a useful tool in relocating a surgical field after a surgical task is completed to perform one or more of the previously described tests on the treated tissue. It is axiomatic that strict sterilization of the operating room and surgical equipment is required during any surgery. The strict hygiene and sterilization conditions required in a "surgical theater," i.e., an operating or treatment room, necessitate the highest possible sterility of all medical devices and equipment. Part of that sterilization process is the need to sterilize anything that comes in contact with the patient or penetrates the sterile field, including the imaging device and its attachments and components. It will be appreciated that the sterile field may be considered a specified area, such as within a tray or on a sterile towel, that is considered free of microorganisms, or the sterile field may be considered an area, immediately around a patient, who has been prepared for a surgical procedure. The sterile field may include the scrubbed team members, who are properly attired, and all furniture and fixtures in the area.

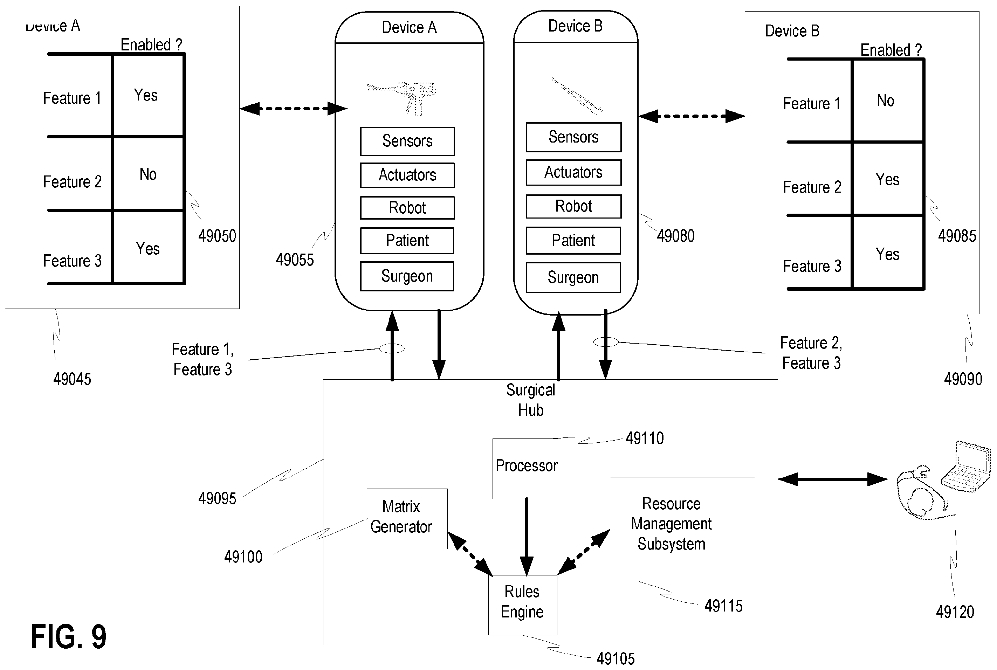

Surgical instrument A 49005 and/or surgical instrument B 49035 may comprise one or more capabilities. (e.g., surgical instrument A comprises capabilities B, Z, and D and surgical instrument comprises capabilities C, F, and E). The capabilities may be associated with features (e.g., A features 49010 associated with surgical instrument A 49005 and B features 49030 associated with surgical instrument B 49035) that the surgical instrument is capable of performing. Examples of the features may be described under the heading "Surgical Instrument Hardware" and in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, for example. For example, if the surgical instrument is an endocutter, one of the capabilities may be resecting tissue (e.g., tissue surrounding a colon when the surgeon in performing a colorectomy). The capabilities may comprise surgical tasks as described with respect to FIG. 9. For example, the capability of resecting tissue may comprise controlling an energy source, cutting, stapling, knob orientation, body orientation, body position, anvil jaw force, reload alignment slot management, and/or the like.

Data may be generated (e.g., by a monitoring module located at the surgical hub 49040 or locally by the surgical instrument) based on the performance of surgical instrument A 49005 and/or B 49035. The data may be relevant to how the surgical instrument is operating is doing in terms of performance. For example, the data may be associated with physical measurement physiological measurements, and/or the like. The measurements are described in greater detail under the heading "Monitoring Of Adjusting A Surgical Parameter Based On Biomarker Measurements" in U.S. Patent Application No. US 17/156, 28, filed November 10, 2021.

An indication of the data may be transmitted to the surgical hub 49040, for example, where it may be evaluated. In examples, the indication may be transmitted to a cloud service (e.g., amazon web services) as described herein. The data may be used as input in an analysis module.

FIG. 9 shows an example of an overview of an adapting interconnected surgical system. As shown in FIG. 9, one or more surgical instruments may be used to perform a surgical task. For example, device A 49045 may be associated with a surgical instrument. Device B 49090 may be associated with a second surgical instrument. The surgical instruments may include one or more features. For example, device A 49045 and/or B 49090 may be associated with one or more features (e.g., device A features 1, 2, and 3 49050 and device B features 1, 2, and 3 49085). The one or more features may be features that are to be performed autonomously (e.g., during the surgery). For example, a feature of device A 49045 may be the orientation of device A 49045. If this feature is enabled, as shown in FIG. 9, the orientation of device A 49045 may be determined (e.g., controlled) autonomously by the system.

If a feature is not enabled, the feature may be performed manually by a surgeon. A feature associated with device B 49090 may be clamping. If this feature is enabled, clamping for device B 49090 may be performed autonomously. If this feature is not enabled, clamping may be performed by a surgeon performing the surgical task. Enabling and disabling the one or more features associated with each of device A 49045 and device B 49090 may be dynamic. For example, while transitioning stages during the performance of a surgical task, the surgical hub 49095 may disable and/or enable one or more of the features associated with device A 49045 and/or device B 49090 based on an introduction or removal of surgical instruments into the surgical OR or based on an introduction of a new surgical system into the surgical OR.

Enabling and/or disabling may be determined based on metrics (e.g., device A metrics 49055 and device B metrics 49080) of the surgical instruments (e.g., how device A 49045 performed its feature, whether it is enabled and on autonomous mode or disabled and on manual mode). In such a case, the metrics may be generated based on one or more of the following sources, which may be located on the surgical instrument: sensors, actuators, robotic metrics, patient biomarkers, force, surgeon biomarkers, surgical context, number of staff, change in number of staff, etc. The patient and/or surgeon biomarkers may be associated with measurements taken from wearable devices in which the patient and/or surgeon may wear during the performance of the surgical task. The actuators may include one or more servomotors located on the surgical instrument. The metrics data associated with these categories may be transmitted to the surgical hub 49095 via a message (e.g., JSON message). The message may pass through a surgical application interface. The surgical hub 49095 may receive this data and may process the data via the surgical hub processor 49110.

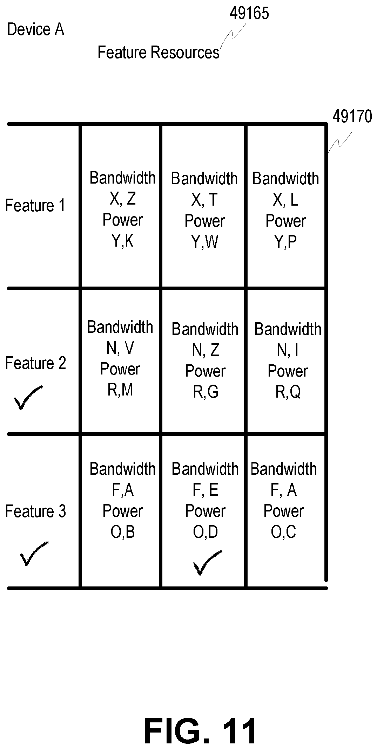

The one or more features may be enabled and/or based on available resources associated with the system. For example, the available resources may be stored in a resource management subsystem 49115 of the surgical hub. Device A 49045 and device B 49090 may use resources from the resource management subsystem 49115 to perform one or more of their features (e.g., autonomously). In examples, the surgical hub 49095 may determine which of the features to enable for the surgical instruments based on the resources available. The surgical hub 49095 may query the resource management subsystem 49115 in order to determine the resources available (e.g., total resources available), which may be described with respect to FIGs. 5 and 6. Determining which features to enable may involve the use of a rules engine 49105. The rules engine 49105 may be located within the surgical hub 49095. In examples, the rules engine 49105 may be located on a third party service. The features may be gathered by the surgical hub 49095 and used to generate a matrix (e.g., using a matrix generator 49100), as described with respect to FIG. 11.

For example, feature 1 of device A 49045 may be associated with (e.g., use) a certain amount of bandwidth and a certain amount of power. This information may be used by the surgical hub 49095 in order to generate a matrix, which may be, for example, a data structure that links the features of each surgical device to the resources (e.g., resource ranges) needed in order to perform the feature. The rules engine 49105 may be responsible for assessing different variations of allocating resources to features and may optimize which features to enable based on an evaluation at the surgical hub 49095. The features that are determined to be enabled may be sent to the surgical instruments via a message. In examples, the resources and/or features of the surgical instruments, may be manually inputted by medical staff member 49120.

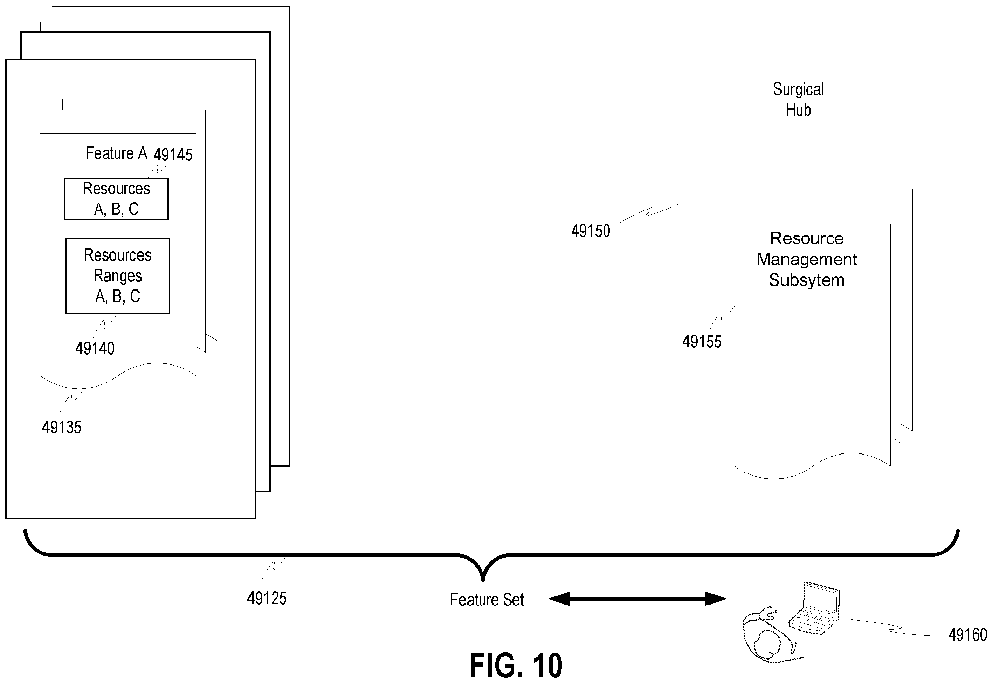

FIG. 10 shows an example of the relationship between feature sets 49125, surgical hub 49150, and resource management. A feature set 49125 may be included in the system. A feature set 49125 may be manually inputted by a user 49160, such as a surgeon or medical staff member. In examples, the feature set 49125 may be a learned set determined using a machine learning algorithm.

One more features may be associated with a surgical instrument. For example, as described with respect to FIG. 2, the one or more features may be functions that the surgical instrument may be able to perform autonomously. If the feature is enabled, this may suggest that the surgical instrument performs this function autonomously, for example, clamping.

The one or more features may be associated with one or more resources 49145. For example, feature A 49135 may be associated with resources 49145 A, B, and C. In examples, the features may be associated with resource ranges 49140. The resource range 49140 may be a range at which the surgical instrument is able to perform the function autonomously (e.g., the range may be include a value indicating an expected performance of the function, which may be based on historical data and surgical context). For example, there may be a minimum resource and maximum resource. In examples, there may be an optimal resource.

The one or more features which are linked to resources 49145 and/or resource ranges 49140 may be transmitted to a surgical hub 49150. The surgical hub 49150 may store and analyze such values in a resource management subsystem 49155, which may include one or more different data structures to hold and to query the features, the resources 49145, and the resource ranges 49140. The features which are enabled may be adjusted (e.g., by changing an autonomy level associated with the feature) based on introduction or removal of one or more surgical instruments or surgical systems. A higher level of autonomy may be associated with a more tasks of the feature being performed autonomously and a lower level of autonomy may be associated with less tasks of the feature being performed autonomously.

In examples, a second surgical hub may be introduced into the surgical OR. The second surgical hub may provide more allowable resources 49145 to be used by the surgical instruments, e.g., already present in the surgical OR. In such a case, more features may be enabled due to the increased allowable resources 49145 as determined by the rules engine of the surgical hub 49150.

Making the determination whether to enable and/or disable one or more features may be performed by the rules engine, as described with respect to FIG. 6. As the instrument (e.g., new instrument) or system (e.g., new system) is introduced, it may register with the surgical hub 49150. The surgical hub 49150 may configure the system or surgical instrument and may notify the other surgical instruments and/or systems already present in the surgical OR. The surgical instruments may have an identification associated with it, as well as the surgical systems, which may be assigned by the surgical hub 49150 at the time of registration.

A device (e.g., new device) and/or system may request (e.g., send a request message) to the surgical hub 49150 asking for permission to connect. The request message may indicate which resources 49145 a system (e.g., new system) has available to be used. A surgical instrument (e.g., new surgical instrument) may send a request message to the surgical hub 49150 asking to connect. The surgical instrument may indicate in the request message which features it is able to do autonomously along with the number of resources 49145 for the features.

The hub 49150 may determine, based on the current setup, whether to allow the surgical system and/or new surgical instrument to connect. After connected, the surgical hub 49150 may make a determination whether to adjust which features are enabled and/or disabled for the current system and may send such determination via a message to the surgical instruments. Based on receiving the message, surgical instruments may follow through and enable and/or disable the features indicated by the surgical hub 49150.

Enabling and/or disabling features may take into account performance metrics, such as latency. The surgical hub 49150 may include an optimization module, which may run cost analysis by enabling and/or disabling a variation of the features in determining an optimal feature set to use for the surgical task at hand.

FIG. 11 shows an example of a matrix generator 49170 and feature determination. As shown in FIG. 11, feature resources 49165 may be associated with a surgical instrument, for example, device A. Feature resources 49165 may be associated with the matrix generated with respect to FIG. 9. For example, feature resources 49165 may show the matrix that was generated, which shows the link between features of device A and resources, as shown in FIG. 11 (e.g., feature one of device A). As the device is connected to the surgical hub, the device may send the features along with the resources associated with the device to the surgical hub as described with respect to FIG. 10. The resources may be the resources used in order to perform the feature of the surgical instrument autonomously. As shown in FIG. 11, feature one of device A may be associated with bandwidth X. The matrix generator 49170 may combine the resources of a secondary device introduced into the surgical hub and run an optimization technique to figure out which features should be enabled in order to best perform the surgical task at hand.

For example, device A feature one may be associated with bandwidth resource (e.g., associated with bandwidth, power, payload effort, size of data packet, transmit frequency, etc.) X and the matrix generator 49170 may input device B's bandwidth of Z and the rules engine (e.g., which obtains this combination from the matrix generator) may determine a cost of enabling feature one with this combination. The matrix generator 49170, in a second variation, may use feature one's bandwidth of X with another device with bandwidth of T and the rules engine may determine a cost associated with this combination. In examples, the matrix generator 49170 may use feature one's bandwidth of X to be combined with a new device introduced into OR which has a bandwidth of L and the rules engine may determine a cost of this combination. After assessing these different variations, the surgical hub may determine (e.g., via the rules engine) whether to enable feature one with a particular tested combination of resources based on a cost analysis, which may be included in an analysis module. If a surgical system is introduced into the OR, feature one's bandwidth of X may be used in a matrix generator 49170 along with a bandwidth (e.g., total bandwidth) of the surgical system, which may be used to determine whether feature one may be enabled.

As shown in FIG. 11, another resource that may be used to determine whether features one, two, or three are enabled may be power. Feature one of device A may be held constant while introduced devices associated with power resources may be used in the matrix generator 49170 and a cost associated with each combination may be determined and used to determine whether feature one should be enabled. This technique may be used for features two and three. For example, in order to perform feature two autonomously for device A, it may use a bandwidth of N. The matrix generator 49170 may hold the bandwidth constant while combining different variations of other devices' bandwidth to determine whether feature two should be enabled. After the features are determined by the surgical hub or a third-party service, the surgical hub may send a message to each of the surgical instruments on which features to enable and may send a notification to a user (e.g., surgeon). In examples, the surgical hub may send a message to a user telling them to disable one or more features manually based on the introduction or removal of a surgical instrument or a surgical system.

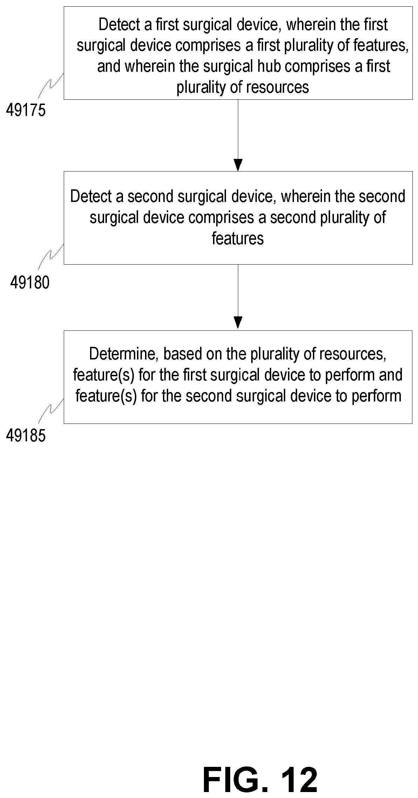

The cost analysis performed by the rules engine may include a measure of the performance of the surgical task. For example, the surgical hub may run a simulation (e.g., locally or remotely via a cloud service) with each of the resource combinations generated by the matrix. Simulation framework may be described in "Method for Surgical Simulation" in U.S. Patent Application No. US 17/332, 593, filed May 27, 2021. The performance metrics may be gathered based on the simulation. For example, a CPI may be determined as well as a latency and throughput. An overall score may be generated for each of the combinations and the surgical hub may determine the maximum score and use the combination of features that resulted in the maximum score for the surgical task simulation. In examples, determining which combination should be used may involve a rules engine assigning weights to performance metrics and other considerations associated with the surgical task. Determining the rules may be based on one more of the following: surgical context, type of features, historical data, or types of resources, as described with respect to FIG. 12. For example, situational awareness may provide the system with additional context not privy to a device (e.g., each device) which may allow the system to optimally adapt. Having awareness of surgical context may provide advantage for the system to adjust appropriately. Historical data may show procedure risk is higher for a certain patient. Historical data may include global and/or patient specific information.

FIG. 12 shows a flow chart of an adapted interconnected surgical system. A surgical hub may be used for autonomous determination of surgical features in a surgical environment. At 49175, the surgical hub may (e.g., include a processor configured to) detect a first surgical device in the surgical environment. The first surgical device may include a first plurality of features. The surgical hub may include a plurality of resources.

At 49180, the surgical hub may detect a second surgical device in the surgical environment. The second surgical device may include a second plurality of features.

At 49185, one or more features may be determined from the first plurality of features for the first surgical device to perform based on the plurality resources. One or more features may be determined from the second plurality of features for the second surgical device to perform based on the plurality of resources.

In examples, the surgical hub may include a plurality of ports. The first surgical device may link to the surgical hub via a first port from the plurality of ports. The second surgical device may link to the surgical hub via a second port form the plurality of ports.

Determining the one or more features from the first plurality of features for the first surgical device to perform and the one or more features from the second plurality of features for the second surgical device to perform may involve using optimization (e.g., Newtonian method) associated with one or more rules. The optimization associated with one or more rules may be based on one or more of the following: surgical context, type of the first plurality of features, type of second plurality of features, historical data, or the plurality of resources.

In example, the surgical hub may determine a surgical context associated with the surgical environment. The surgical hub may determine the one or more features from the first plurality of features for the first surgical device to perform and the one or more features from the second plurality of features for the second surgical device to perform based on the plurality of resources and the surgical context.

The surgical hub may send an indication of the determined one or more features to a user of the first surgical device and a user of the second surgical device. The surgical hub may detect a third surgical device in the surgical environment. The third surgical device may include a third plurality of features. The surgical hub may adjust the one or more features from the first plurality of features for the first surgical device to perform and the one or more features from the second plurality of features for the second surgical device to perform based on the plurality of resources and the third plurality of features. One or more feature from the third plurality of features for the third surgical device to performed may be determined based on the plurality of resources.

The surgical hub may determine one or more optional features from the first plurality of features for the first surgical device to perform and one or more optional features from the second plurality of features for the second surgical device to perform based on the plurality of resources. The optional features may be selected by a user of the first surgical instrument and a user of the second surgical instrument. The surgical hub may determine one or resources from the plurality of resources for the first surgical device and one or resources from the plurality of resources for the second surgical device. The determined one or more resources may be used to perform the determine one or more features.

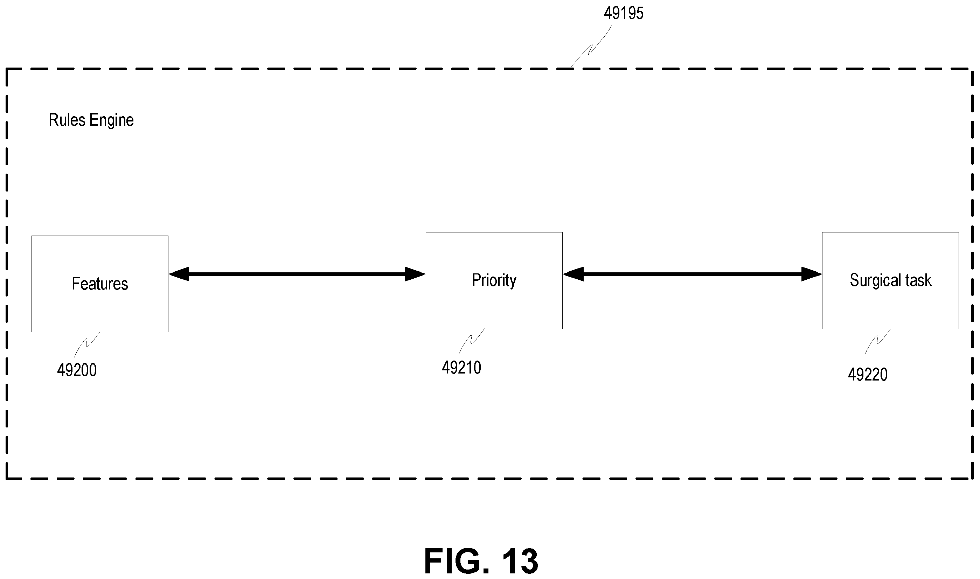

FIG. 13 shows an example of an overview of a rules engine 49195. In examples, a rules engine may 49195 be included in a third-party system, which the surgical hub may have access to (e.g., using a security key). The rules engine 49195 may be linked to the matrix generator as described with respect to FIG. 9. The rules engine may assign weights based on the importance of the features 49200 of the surgical instrument that may be performed autonomously, as well as the resources associated with the surgical instruments. For example, feature one as described with respect to FIG. 11 may be given a higher weight when compared to feature two. Feature two as described with respect to FIG. 11 of device A may be given a higher weight than feature three. The weights may be determined based on surgical context and/or historical data. The weights maybe determined based on results gathered from surgical simulations.

Weight may be assigned to performance metrics. Weights may be assigned to resources for each of the features 49205. For example, for feature one, as described with respect to FIG. 11, bandwidth may be assigned a higher weight when compared to power. As described with respect to FIG. 11, power for feature two of device A maybe assigned a higher weight than bandwidth. This may depend on the type of feature 49200 that is being performed autonomously. For example, power may be assigned a higher weight for performing orientation autonomously when compared to the power used to perform clamping autonomously.

Based on the weights, a priority 49210 may be assigned to the resources associated with each of the features 49200. A high and/or low priority 49210 may be determined. A feature 49200 associated with a high priority 49210 may be chosen to be enabled over a feature 49200 associated with a low priority 49210. As the surgical task 49220 is performed and is executed, the weights may be dynamic based on surgical context. The rules engine 49195 may determine update weights for each of the features 49200 of the surgical instruments. The weights may be updated based on a new surgical instrument or new system being introduced into the surgical OR.

Autonomous system interactions may be provided. Adaptation of the autonomous options and/or processes may be based on the automatic identification and/or connection in between the system and secondary systems. Adaptation of automated functional options may be based on the interconnections of the systems. For example, the surgical hub may connect to one or more secondary systems and in establishing connections may adjust the operations that may be conducted autonomously. The adjustments may include setup, updating operational systems, designating communication priorities or processor load priorities, and/or establishing communication between secondary systems (e.g., two secondary systems) directly.

Autonomous communication establishment and/or registration may be provided. The surgical hub and/or instrument may be associated with a setup and/or a configuration at startup or access to a smart network. The surgical hub and/or or instrument setup and/or configuration at startup or access to a smart network may be described herein. In examples, if a device requests and/or establishes a connection to the hub defined network, the hub may define for the device a type of communication, frequency, system security level (e.g., causing the device to adjust its security level), flagging technique, revisions of software, etc. During this defining of communication exchange, the hub, based on the procedure, network backlog/latency, capacity level, and/or procedure, may adjust the anticipated intercommunication levels to the situation it is monitoring. The hub may define that multiple systems within its network communicate directly (e.g., using network bandwidth) and/or communicate through the hub (e.g., allowing it to record and adapt communication based on additional data), which may depend on the capabilities and capacities of the hub at the time and for the specific procedure. For example, the hub may determine the mode (e.g., best mode) of communications between devices (e.g., two devices) and may tell the devices how to communicate with each other. The hub may send flagging information to the devices to check back in with the Hub, for example, based on a certain change. The hub may request that the device adjusts the frequencies it operates on, the magnitude the system communicates at (e.g., in dB), and/or the protocols it uses based on the amount of noise and/or data detected within the OR by the hub. The hub may adjust numerous systems communication instructions based on the introduction of a device, the priority of its data or risk in its function relative to other systems within the network, to provide the highest priority or highest risk the data that uses the most capacity for interaction. It may define the communication interactions between systems (e.g., two systems) within its system to tune their communication. For example, the hub may detect a device's signal is decreasing or that the next step in the procedure uses a higher bandwidth to communicate the appropriate amount of data. The hub may enable an antenna (e.g., additional antenna) to try and increase the signal strength or it may tell the device to increase the signal output strength.

Connecting to an available network and/or requesting registration as a participant in the hub control matrix may be provided. Automated identification (ID) and/or registration may be based on detected aspects of the network and/or device. Unlinked and connectable surgical devices may be aware of the networks surrounding it. The awareness may allow for a selective interrogation of the possible connection options. The options may be compared to a pre-existing table or hierarchal order of networks it should have a preference for connecting to. The device may announce its intentions to connect and the network may provide information relevant to which of the networks are anticipating its arrival and/or of the ones that do not want it to interfere or try to connect to. This may be based on the procedure step, procedure progress, instruments in communication (e.g., currently in communications), arrival of duplicate devices or incompatible devices, and/or another situational use. Automated adaptation of a system function may be based on a parameter of the establishment of communication between systems. The parameter may be a step in the procedure. For example, on establishing the connection, the device may adapt the system if (e.g., only if) it occurs within a certain step in the procedure.

Automatically changing and/or updating software and/or functional testing the assembled configuration (e.g., after first assembly) may be used to confirm adaptation. The software may update the configuration settings. A validation of the update may be used. Functional check of the configuration may include one or more of the following. It may include verification of transducer function. It may include verification of torque. It may include articulation angle maximums. It may include re-establishing the device zero position and/or home. Shipping issues may cause the device to fall out of calibrations. For example, an energy device may be announced and/or detected by the hub. The hub may know the next step and/or use case (e.g., based on factory set configurations, surgeon selected/modified/machine, or clinical database learning) for the announced device in the procedure. The hub may automatically send configuration settings to the device for its activation.

Selective automatic establishment of communication between systems within the same operating room (OR) network may be provided, which may include distributed analysis, communication and data verification by co-networked OR devices. For example, interactive communication of operating parameters between a device and a hub controlling the procedure plan and/or flow of the surgery may include one or more of the following. A surgical stapling device may be preset at the factory to 15mm/sec cutting speed by default. The stapler may be energized within the OR and may establish communication with the hub. The hub may recognize the device. The hub may analyze a database and determine that the optimal speed for this procedure is 5mm/sec and a 20 second precompression delay. The hub may communicate to the device and send updates of the cutting speed and compression times. The device may use the parameters and check that the values are within the operation boundaries of the device system. The device may communicate back to hub that 5mm/sec is on the lower bound of the systems motor performance, which may potentially result an insufficient sealing staple line delivery and/or device performance degradation. The hub may take the updated information and determine a risk profile at higher cutting speeds. An acceptable increase may be determined and passed back to the device. The device may verify the parameters and acknowledge to the HUB that it is ready. One or more of the techniques described herein may done autonomously. If an acceptable solution cannot be achieved, the HUB may alert the OR personal. Based on the risk levels of the patient, device and procedure, the system may set an overall procedure risk level. Depending on the calculated risk level, the system may complete the task autonomously or if the risk is deemed too high, the system may wait to be acknowledged by the surgeon before completing the procedure step. A mobile flexible endoscopic robotic may be rolled into the OR for a portion of trans-bronchial imaging and dissections and/or mobilization. If the system becomes aware it is operating in close proximity with the patient of other smart or digital eco-system device (e.g., lap retractors, staples, energy devices, etc.), it may establish communication with the devices when it controls the system close to the other systems to help avoid collisions or inadvertent tissue tension situations.

Autonomous identification of trackable and/or identifiable elements may be within system range. Automatic identification of wearable systems may include one or more of the following. It may include automatic determination of an active user of the wearable, which may involve using previous datasets to determine the user and/or automated verification and/or confirmation of identify (e.g., use of fingerprint of previous biomarker to verify the user). It may include categorization of user's characteristics such as job (e.g., skillset, competency level, equipment trained on, etc.), determination of use of monitored data, and/or prioritization of monitored data relative to other data sources simultaneously processed by the system. Automatic defining of reporting frequency, registration, and/or data collection parameters. Instrument, tool, medical stock identification may be provided, which may include detection of make, model, and/or serial number. Auto-determination of system limits or reach may include which systems the hub is allowed to control, acceptable reach for communication establishment, and/or physical and/or functional boundaries to track ability.

## Claims

1. A surgical hub (49095) for autonomous determination of surgical device features to be enabled for a surgical procedure, the surgical hub comprising:
a processor configured to:
detect a first surgical device (49045) in the surgical environment, wherein the first surgical device (49045) comprises a first plurality of features (49050), and wherein the surgical hub (49095) comprises a plurality of resources;
detect a second surgical device (49090) in the surgical environment, wherein the second surgical device (49090) comprises a second plurality of features (49085);
receive or derive an indication of the surgical procedure to be performed, the surgical procedure comprising a plurality of surgical tasks; and
determine one or more features from the first plurality of features for the first surgical device (49045) to be enabled or disabled to perform a surgical task of the plurality of surgical tasks and determine one or more features from the second plurality of features for the second surgical device (49090) to be enabled or disabled to perform a surgical task of the plurality of surgical tasks,
wherein the features are features that the respective surgical device is capable of performing,
wherein an enabled feature is a feature for performing the surgical task of the plurality of surgical tasks autonomously, and
wherein a disabled feature is a feature for performing the surgical task of the plurality of surgical tasks manually by a surgeon,
wherein the determination of which features to enable or disable is based on the plurality of resources.

2. The surgical hub (49095) of claim 1, wherein the processor is further configured to:
send an indication of the determined one or more features to a user of the first surgical device (49045) and a user of the second surgical device (49090); or
transmit a signal to the first (49045) and/or second (49090) device to enable the determined one or more features.

3. The surgical hub (49095) of claim 1, wherein the first (49045) and/or the second (49090) device is an endocutter and the first and/or second plurality of features comprises one or more of the following: controlling an energy source, cutting, stapling, knob orientation, body orientation, body position, clamping, anvil jaw force, reload alignment slot management.

4. The surgical hub (49095) of any preceding claim, wherein the processor is further configured to receive or determine an indication of the first and second plurality of features.

5. The surgical hub (49095) of any preceding claim, wherein the processor is configured to detect the first (49045) and second (49090) devices when the first (49045) and second (49090) devices send a request to couple with the hub (49095), or when they couple to the hub (49095).

6. The surgical hub (49095) of any preceding claim, wherein the surgical hub (49095) comprises a plurality of ports, and wherein the first surgical device (49045) links to the surgical hub (49095) via a first port from the plurality of ports, and wherein the second surgical device (49090) links to the surgical hub (49095) via a second port form the plurality of ports.

7. The surgical hub (49095) of any preceding claim, wherein determining which features should be enabled comprises using optimization associated with one or more rules,
optionally wherein the optimization associated with one or more rules is based on at least one of: surgical context, type of the first plurality of features, type of second plurality of features, historical data, or the plurality of resources.

8. The surgical hub (49095) of any preceding claim, wherein the processor is further configured to:
detect a third surgical device in the surgical environment, wherein the third surgical device comprises a third plurality of features; and
adjust the one or more features from the first plurality of features for the first surgical device (49045) to perform and the one or more features from the second plurality of features for the second surgical device (49090) to perform based on the plurality of resources and the third plurality of features,
optionally wherein the adjustment comprises disabling a feature that was previously enabled.

9. The surgical hub (49095) of any preceding claim, wherein the processor is further configured to:
detect a third surgical device in the surgical environment, wherein the third surgical device comprises a third plurality of features; and
determine one or more features from the third plurality of features for the third surgical device to perform based on the plurality of resources.

10. The surgical hub (49095) of any preceding claim, wherein the processor is further configured to:
determine one or more optional features from the first plurality of features for the first surgical device (49045) to perform and one or more optional features from the second plurality of features for the second surgical device (49090) to perform based on the plurality of resources,
optionally wherein the optional features are selected by a user of the first surgical instrument and a user of the second surgical instrument.

11. A method for autonomous determination of surgical device features to be enabled for a surgical procedure, the method comprising:
detecting a first surgical device (49045) in the surgical environment, wherein the first surgical device (49045) comprises a first plurality of features, and wherein a surgical hub (49095) comprises a plurality of resources;
detecting a second surgical device (49090) in the surgical environment, wherein the second surgical device (49090) comprises a second plurality of features;
receiving or deriving an indication of the surgical procedure to be performed, the surgical procedure comprising a plurality of surgical tasks; and
determining one or more features from the first plurality of features for the first surgical device (49045) to be enabled or disabled to perform a surgical task of the plurality of surgical tasks and determining one or more features from the second plurality of features for the second surgical device (49090) to be enabled or disabled to perform a surgical task of the plurality of surgical tasks,
wherein the features are features that the respective surgical device is capable of performing,
wherein an enabled feature is a feature for performing the surgical task of the plurality of surgical tasks autonomously, and
wherein a disabled feature is a feature for performing the surgical task of the plurality of surgical tasks manually by a surgeon,
wherein the determination is based on the plurality of resources.

12. The method of claim 11, further comprising:
sending an indication of the determined one or more features to a user of the first surgical device (49045) and a user of the second surgical device (49090); or
transmit a signal to the first (49045) and/or second (49090) device to enable the determined one or more features.

13. The method of claim 11, wherein the first (49045) and/or the second (49090) device is an endocutter and the plurality of first and/or second features comprises one or more of the following: controlling an energy source, cutting, stapling, knob orientation, body orientation, body position, clamping, anvil jaw force, reload alignment slot management.

14. The method of any one of claims 11-13, further comprising receiving or determining an indication of the first and second plurality of features.

15. The method of any one of claims 11-14, wherein detecting the first (49045) and second (49090) devices comprises detecting when the first (49045) and second (49090) devices send a request to couple with the hub (49090), or detecting when they have been coupled to the hub (49095).

16. The method of any one of claims 11-15, wherein the surgical hub (49095) comprises a plurality of ports, and wherein the first surgical device (49045) links to the surgical hub (49095) via a first port from the plurality of ports, and wherein the second surgical device (49090) links to the surgical hub (49095) via a second port form the plurality of ports.

17. The method of any one of claims 11-16, wherein determining which features should be enabled comprises using optimization associated with one or more rules,
optionally wherein the optimization associated with one or more rules is based on at least one of: surgical context, type of the first plurality of features, type of second plurality of features, historical data, or the plurality of resources.

18. The method of any one of claims 11-17, further comprising:
detecting a third surgical device in the surgical environment, wherein the third surgical device comprises a third plurality of features; and
adjusting the one or more features from the first plurality of features for the first surgical device (49045) to perform and the one or more features from the second plurality of features for the second surgical device (49090) to perform based on the plurality of resources and the third plurality of features,
optionally wherein the adjustment comprises disabling a feature that was previously enabled.

19. The method of any one of claims 11-18, further comprising:
detecting a third surgical device in the surgical environment, wherein the third surgical device comprises a third plurality of features; and
determining one or more features from the third plurality of features for the third surgical device to perform based on the plurality of resources.

20. The method of any preceding claim, further comprising:
determining one or more optional features from the first plurality of features for the first surgical device to perform and one or more optional features from the second plurality of features for the second surgical device to perform based on the plurality of resources,
optionally wherein the optional features are selected by a user of the first surgical instrument and a user of the second surgical instrument.

21. A computer program comprising instructions which, when the program is executed by a computer, cause the computer to carry out the method of any of claims 11 to 20.

22. A computer-readable medium comprising instructions which, when executed by a computer, cause the computer to carry out the method of any of claims 11 to 20.

## Patentansprüche

1. Chirurgische Nabe (49095) zur autonomen Bestimmung von Merkmalen einer chirurgischen Vorrichtung, die für einen chirurgischen Eingriff aktiviert werden sollen, die chirurgische Nabe umfassend:
einen Prozessor, der konfiguriert ist zum:
Erkennen einer ersten chirurgischen Vorrichtung (49045) in der chirurgischen Umgebung, wobei die erste chirurgische Vorrichtung (49045) eine erste Vielzahl von Merkmalen (49050) umfasst, und wobei der chirurgische Knotenpunkt (49095) eine Vielzahl von Ressourcen umfasst;
Erkennen einer zweiten chirurgischen Vorrichtung (49090) in der chirurgischen Umgebung, wobei die zweite chirurgische Vorrichtung (49090) eine zweite Vielzahl von Merkmalen (49085) umfasst;
Empfangen oder Ableiten einer Angabe über die durchzuführende chirurgische Prozedur, wobei die chirurgische Prozedur eine Vielzahl von chirurgischen Aufgaben umfasst; und
Bestimmen eines oder mehrerer Merkmale aus der ersten Vielzahl von Merkmalen der ersten chirurgischen Vorrichtung (49045), die für die Durchführung einer chirurgischen Aufgabe aus der Vielzahl von chirurgischen Aufgaben aktiviert oder deaktiviert werden sollen, und Bestimmen eines oder mehrerer Merkmale aus der zweiten Vielzahl von Merkmalen der zweiten chirurgischen Vorrichtung (49090), die für die Durchführung einer chirurgischen Aufgabe aus der Vielzahl von chirurgischen Aufgaben aktiviert oder deaktiviert werden sollen,
wobei die Merkmale Merkmale sind, die die jeweilige chirurgische Vorrichtung ausführen kann,
wobei ein aktiviertes Merkmal ein Merkmal zur autonomen Durchführung der chirurgischen Aufgabe aus der Vielzahl der chirurgischen Aufgaben ist, und
wobei ein deaktiviertes Merkmal ein Merkmal zur manuellen Durchführung der chirurgischen Aufgabe aus der Vielzahl der chirurgischen Aufgaben durch einen Chirurgen ist,
wobei die Bestimmung, welche Merkmale zu aktivieren oder zu deaktivieren sind, auf der Vielzahl von Ressourcen basiert.

2. Chirurgische Nabe (49095) nach Anspruch 1, wobei der Prozessor ferner konfiguriert ist zum:
Senden einer Angabe des bestimmten einen oder der mehreren Merkmale an einen Benutzer der ersten chirurgischen Vorrichtung (49045) und einen Benutzer der zweiten chirurgischen Vorrichtung (49090); oder
Übertragen eines Signals an die erste (49045) und/oder zweite (49090) Vorrichtung, um das bestimmte eine oder die mehreren Merkmale zu aktivieren.

3. Chirurgische Nabe (49095) nach Anspruch 1, wobei die erste (49045) und/oder die zweite (49090) Vorrichtung ein Endocutter ist und die erste und/oder zweite Vielzahl von Merkmalen eines oder mehrere von Folgendem umfasst: Steuern einer Energiequelle, Schneiden, Heften, Knopfausrichtung, Körperausrichtung, Körperposition, Klemmen, Ambossbackenkraft, Nachladeausrichtung, Schlitzverwaltung.

4. Chirurgische Nabe (49095) nach einem der vorstehenden Ansprüche, wobei der Prozessor ferner konfiguriert ist, um eine Angabe der ersten und zweiten Vielzahl von Merkmalen zu empfangen oder zu bestimmen.

5. Chirurgische Nabe (49095) nach einem der vorstehenden Ansprüche, wobei der Prozessor konfiguriert ist, um die erste (49045) und die zweite (49090) Vorrichtung zu erkennen, wenn die erste (49045) und die zweite (49090) Vorrichtung eine Anfrage zum Koppeln mit der Nabe (49095) senden, oder wenn sie mit der Nabe (49095) koppeln.

6. Chirurgische Nabe (49095) nach einem der vorstehenden Ansprüche, wobei die chirurgische Nabe (49095) eine Vielzahl von Anschlüssen umfasst und wobei die erste chirurgische Vorrichtung (49045) über einen ersten Anschluss aus der Vielzahl der Anschlüsse mit der chirurgischen Nabe (49095) verbunden ist und wobei die zweite chirurgische Vorrichtung (49090) über einen zweiten Anschluss aus der Vielzahl der Anschlüsse mit der chirurgischen Nabe (49095) verbunden ist.

7. Chirurgische Nabe (49095) nach einem der vorstehenden Ansprüche, wobei das Bestimmen, welche Merkmale aktiviert werden sollen, die Verwendung einer Optimierung in Verbindung mit einer oder mehreren Regeln umfasst,
optional wobei die mit einer oder mehreren Regeln verbundene Optimierung mindestens auf einem von Folgendem basiert: chirurgischer Kontext, Typ der ersten Vielzahl von Merkmalen, Typ der zweiten Vielzahl von Merkmalen, historische Daten oder die Vielzahl von Ressourcen.

8. Chirurgische Nabe (49095) nach einem der vorstehenden Ansprüche, wobei der Prozessor ferner konfiguriert ist zum:
Erkennen einer dritten chirurgischen Vorrichtung in der chirurgischen Umgebung, wobei die dritte chirurgische Vorrichtung eine dritte Vielzahl von Merkmalen umfasst; und
Anpassen des einen oder der mehreren Merkmale aus der ersten Vielzahl von Merkmalen, die die erste chirurgische Vorrichtung (49045) ausführen soll, und des einen oder der mehreren Merkmale aus der zweiten Vielzahl von Merkmalen, die die zweite chirurgische Vorrichtung (49090) ausführen soll, basierend auf der Vielzahl von Ressourcen und der dritten Vielzahl von Merkmalen,
optional wobei die Anpassung die Deaktivierung eines Merkmals umfasst, das zuvor aktiviert war.

9. Chirurgische Nabe (49095) nach einem der vorstehenden Ansprüche, wobei der Prozessor ferner konfiguriert ist zum:
Erkennen einer dritten chirurgischen Vorrichtung in der chirurgischen Umgebung, wobei die dritte chirurgische Vorrichtung eine dritte Vielzahl von Merkmalen umfasst; und
Bestimmen eines oder mehrerer Merkmale aus der dritten Vielzahl von Merkmalen, die die dritte chirurgische Vorrichtung basierend auf der Vielzahl von Ressourcen ausführen soll.

10. Chirurgische Nabe (49095) nach einem der vorstehenden Ansprüche, wobei der Prozessor ferner konfiguriert ist zum:
Bestimmen eines oder mehrerer optionaler Merkmale aus der ersten Vielzahl von Merkmalen, die die erste chirurgische Vorrichtung (49045) ausführen soll, und eines oder mehrerer optionaler Merkmale aus der zweiten Vielzahl von Merkmalen, die die zweite chirurgische Vorrichtung (49090) ausführen soll, basierend auf der Vielzahl von Ressourcen,
optional wobei die optionalen Merkmale von einem Benutzer des ersten chirurgischen Instruments und einem Benutzer des zweiten chirurgischen Instruments ausgewählt werden.

11. Verfahren zur autonomen Bestimmung von Merkmalen einer chirurgischen Vorrichtung, die für eine chirurgische Prozedur aktiviert werden sollen, das Verfahren umfassend:
Erkennen einer ersten chirurgischen Vorrichtung (49045) in der chirurgischen Umgebung, wobei die erste chirurgische Vorrichtung (49045) eine erste Vielzahl von Merkmalen umfasst, und wobei ein chirurgischer Knotenpunkt (49095) eine Vielzahl von Ressourcen umfasst;
Erkennen einer zweiten chirurgischen Vorrichtung (49090) in der chirurgischen Umgebung, wobei die zweite chirurgische Vorrichtung (49090) eine zweite Vielzahl von Merkmalen umfasst;
Empfangen oder Ableiten einer Angabe über die durchzuführende chirurgische Prozedur, wobei die chirurgische Prozedur eine Vielzahl von chirurgischen Aufgaben umfasst; und
Bestimmen eines oder mehrerer Merkmale aus der ersten Vielzahl von Merkmalen der ersten chirurgischen Vorrichtung (49045), die für die Durchführung einer chirurgischen Aufgabe aus der Vielzahl von chirurgischen Aufgaben aktiviert oder deaktiviert werden sollen, und Bestimmen eines oder mehrerer Merkmale aus der zweiten Vielzahl von Merkmalen der zweiten chirurgischen Vorrichtung (49090), die für die Durchführung einer chirurgischen Aufgabe aus der Vielzahl von chirurgischen Aufgaben aktiviert oder deaktiviert werden sollen,
wobei die Merkmale Merkmale sind, die die jeweilige chirurgische Vorrichtung ausführen kann,
wobei ein aktiviertes Merkmal ein Merkmal zur autonomen Durchführung der chirurgischen Aufgabe aus der Vielzahl der chirurgischen Aufgaben ist, und
wobei ein deaktiviertes Merkmal ein Merkmal zur manuellen Durchführung der chirurgischen Aufgabe aus der Vielzahl der chirurgischen Aufgaben durch einen Chirurgen ist,
wobei die Bestimmung auf der Vielzahl der Ressourcen basiert.

12. Verfahren nach Anspruch 11, ferner umfassend:
Senden einer Angabe des bestimmten einen oder der mehreren Merkmale an einen Benutzer der ersten chirurgischen Vorrichtung (49045) und einen Benutzer der zweiten chirurgischen Vorrichtung (49090); oder
Übertragen eines Signals an die erste (49045) und/oder zweite (49090) Vorrichtung, um das bestimmte eine oder die mehreren Merkmale zu aktivieren.

13. Verfahren nach Anspruch 11, wobei die erste (49045) und/oder die zweite (49090) Vorrichtung ein Endocutter ist und die Vielzahl der ersten und/oder zweiten Merkmale eines oder mehrere von Folgendem umfasst: Steuern einer Energiequelle, Schneiden, Heften, Knopfausrichtung, Körperausrichtung, Körperposition, Klemmen, Ambossbackenkraft, Nachladeausrichtungsschlitzverwaltung.

14. Verfahren nach einem der Ansprüche 11 bis 13, ferner umfassend das Empfangen oder Bestimmen einer Angabe der ersten und zweiten Vielzahl von Merkmalen.

15. Verfahren nach einem der Ansprüche 11 bis 14, wobei das Erkennen der ersten (49045) und zweiten (49090) Vorrichtung das Erkennen umfasst, wenn die erste (49045) und zweite (49090) Vorrichtung eine Anfrage zum Koppeln mit der Nabe (49090) senden, oder das Erkennen, wenn sie mit der Nabe (49095) gekoppelt wurden.

16. Verfahren nach einem der Ansprüche 11 bis 15, wobei die chirurgische Nabe (49095) eine Vielzahl von Anschlüssen umfasst und wobei die erste chirurgische Vorrichtung (49045) über einen ersten Anschluss aus der Vielzahl der Anschlüsse mit der chirurgischen Nabe (49095) verbunden ist und wobei die zweite chirurgische Vorrichtung (49090) über einen zweiten Anschluss aus der Vielzahl der Anschlüsse mit der chirurgischen Nabe (49095) verbunden ist.

17. Verfahren nach einem der Ansprüche 11 bis 16, wobei das Bestimmen, welche Merkmale aktiviert werden sollen, die Verwendung einer Optimierung in Verbindung mit einer oder mehreren Regeln umfasst,
optional wobei die mit einer oder mehreren Regeln verbundene Optimierung mindestens auf einem von Folgendem basiert: chirurgischer Kontext, Typ der ersten Vielzahl von Merkmalen, Typ der zweiten Vielzahl von Merkmalen, historische Daten oder die Vielzahl von Ressourcen.

18. Verfahren nach einem der Ansprüche 11 bis 17, ferner umfassend:
Erkennen einer dritten chirurgischen Vorrichtung in der chirurgischen Umgebung, wobei die dritte chirurgische Vorrichtung eine dritte Vielzahl von Merkmalen umfasst; und
Anpassen des einen oder der mehreren Merkmale aus der ersten Vielzahl von Merkmalen, die die erste chirurgische Vorrichtung (49045) ausführen soll, und des einen oder der mehreren Merkmale aus der zweiten Vielzahl von Merkmalen, die die zweite chirurgische Vorrichtung (49090) ausführen soll, basierend auf der Vielzahl von Ressourcen und der dritten Vielzahl von Merkmalen,
optional wobei die Anpassung die Deaktivierung eines Merkmals umfasst, das zuvor aktiviert war.

19. Verfahren nach einem der Ansprüche 11 bis 18, ferner umfassend:
Erkennen einer dritten chirurgischen Vorrichtung in der chirurgischen Umgebung, wobei die dritte chirurgische Vorrichtung eine dritte Vielzahl von Merkmalen umfasst; und
Bestimmen eines oder mehrerer Merkmale aus der dritten Vielzahl von Merkmalen für die dritte chirurgische Vorrichtung, die auf der Vielzahl von Ressourcen basiert.

20. Verfahren nach einem der vorstehenden Ansprüche, ferner umfassend:
Bestimmen eines oder mehrerer optionaler Merkmale aus der ersten Vielzahl von Merkmalen, die die erste chirurgische Vorrichtung ausführen soll, und eines oder mehrerer optionaler Merkmale aus der zweiten Vielzahl von Merkmalen, die die zweite chirurgische Vorrichtung ausführen soll, basierend auf der Vielzahl von Ressourcen,
optional wobei die optionalen Merkmale von einem Benutzer des ersten chirurgischen Instruments und einem Benutzer des zweiten chirurgischen Instruments ausgewählt werden.

21. Computerprogramm, umfassend Anweisungen, die, wenn das Programm von einem Computer ausgeführt wird, den Computer veranlassen, das Verfahren nach einem der Ansprüche 11 bis 20 auszuführen.

22. Computerlesbares Medium, umfassend Anweisungen, die, wenn sie von einem Computer ausgeführt werden, den Computer veranlassen, das Verfahren nach einem der Ansprüche 11 bis 20 auszuführen.

## Revendications

1. Concentrateur chirurgical (49095) pour la détermination autonome de caractéristiques de dispositif chirurgical à activer pour une intervention chirurgicale, le concentrateur chirurgical comprenant :
un processeur configuré pour :
détecter un premier dispositif chirurgical (49045) dans l'environnement chirurgical, dans lequel le premier dispositif chirurgical (49045) comprend une première pluralité de caractéristiques (49050), et dans lequel le concentrateur chirurgical (49095) comprend une pluralité de ressources ;
détecter un deuxième dispositif chirurgical (49090) dans l'environnement chirurgical, dans lequel le deuxième dispositif chirurgical (49090) comprend une deuxième pluralité de caractéristiques (49085) ;
recevoir ou déduire une indication de l'intervention chirurgicale à mettre en œuvre, l'intervention chirurgicale comprenant une pluralité de tâches chirurgicales ; et
déterminer une ou plusieurs caractéristiques de la première pluralité de caractéristiques pour le premier dispositif chirurgical (49045) à activer ou à désactiver pour mettre en œuvre une tâche chirurgicale de la pluralité de tâches chirurgicales et déterminer une ou plusieurs caractéristiques de la deuxième pluralité de caractéristiques pour le deuxième dispositif chirurgical (49090) à activer ou à désactiver pour mettre en œuvre une tâche chirurgicale de la pluralité de tâches chirurgicales,
dans lequel les caractéristiques sont des caractéristiques que le dispositif chirurgical respectif est capable de mettre en œuvre,
dans lequel une caractéristique activée est une caractéristique permettant de mettre en œuvre la tâche chirurgicale de la pluralité de tâches chirurgicales de manière autonome, et
dans lequel une caractéristique désactivée est une caractéristique permettant à un chirurgien de mettre en œuvre manuellement la tâche chirurgicale de la pluralité de tâches chirurgicales,
dans lequel la détermination des caractéristiques à activer ou à désactiver est basée sur la pluralité de ressources.

2. Concentrateur chirurgical (49095) selon la revendication 1, dans lequel le processeur est en outre configuré pour :
envoyer une indication de la ou des caractéristiques déterminées à un utilisateur du premier dispositif chirurgical (49045) et à un utilisateur du deuxième dispositif chirurgical (49090) ; ou
transmettre un signal au premier (49045) et/ou au deuxième (49090) dispositif pour activer la ou les caractéristiques déterminées.

3. Concentrateur chirurgical (49095) selon la revendication 1, dans lequel le premier (49045) et/ou le deuxième (49090) dispositif est un dispositif de coupe endoscopique et la première et/ou la deuxième pluralité de caractéristiques comprend l'un ou plusieurs de ce qui suit : commande d'une source d'énergie, coupe, agrafage, orientation de bouton, orientation de corps, position de corps, serrage, force de mâchoire d'enclume, gestion de fente d'alignement de rechargement.

4. Concentrateur chirurgical (49095) selon l'une quelconque revendication précédente, dans lequel le processeur est en outre configuré pour recevoir ou déterminer une indication de la première et de la deuxième pluralité de caractéristiques.

5. Concentrateur chirurgical (49095) selon l'une quelconque revendication précédente, dans lequel le processeur est configuré pour détecter les premier (49045) et deuxième (49090) dispositifs lorsque les premier (49045) et deuxième (49090) dispositifs envoient une demande de couplage avec le concentrateur (49095), ou lorsqu'ils se couplent au concentrateur (49095).

6. Concentrateur chirurgical (49095) selon l'une quelconque revendication précédente, dans lequel le concentrateur chirurgical (49095) comprend une pluralité de ports, et dans lequel le premier dispositif chirurgical (49045) se lie au concentrateur chirurgical (49095) par l'intermédiaire d'un premier port de la pluralité de ports, et dans lequel le deuxième dispositif chirurgical (49090) se lie au concentrateur chirurgical (49095) par l'intermédiaire d'un second port de la pluralité de ports.

7. Concentrateur chirurgical (49095) selon l'une quelconque revendication précédente, dans lequel la détermination des caractéristiques à activer comprend l'utilisation d'une optimisation associée à une ou plusieurs règles,
facultativement dans lequel l'optimisation associée à une ou plusieurs règles est basée sur l'un parmi : contexte chirurgical, type de la première pluralité de caractéristiques, type de la deuxième pluralité de caractéristiques, données historiques, ou la pluralité de ressources.

8. Concentrateur chirurgical (49095) selon l'une quelconque revendication précédente, dans lequel le processeur est en outre configuré pour :
détecter un troisième dispositif chirurgical dans l'environnement chirurgical, dans lequel le troisième dispositif chirurgical comprend une troisième pluralité de caractéristiques ; et
ajuster la ou les caractéristiques de la première pluralité de caractéristiques à mettre en œuvre par le premier dispositif chirurgical (49045) et la ou les caractéristiques de la deuxième pluralité de caractéristiques à mettre en œuvre par le deuxième dispositif chirurgical (49090) sur la base de la pluralité de ressources et de la troisième pluralité de caractéristiques,
facultativement dans lequel l'ajustement comprend la désactivation d'une caractéristique qui était précédemment activée.

9. Concentrateur chirurgical (49095) selon l'une quelconque revendication précédente, dans lequel le processeur est en outre configuré pour :
détecter un troisième dispositif chirurgical dans l'environnement chirurgical, dans lequel le troisième dispositif chirurgical comprend une troisième pluralité de caractéristiques ; et
déterminer une ou plusieurs caractéristiques de la troisième pluralité de caractéristiques à mettre en œuvre par le troisième dispositif chirurgical sur la base de la pluralité de ressources.

10. Concentrateur chirurgical (49095) selon l'une quelconque revendication précédente, dans lequel le processeur est en outre configuré pour :
déterminer une ou plusieurs caractéristiques facultatives de la première pluralité de caractéristiques à mettre en œuvre par le premier dispositif chirurgical (49045) et une ou plusieurs caractéristiques facultatives de la deuxième pluralité de caractéristiques à mettre en œuvre par le deuxième dispositif chirurgical (49090) sur la base de la pluralité de ressources,
facultativement dans lequel les caractéristiques facultatives sont sélectionnées par un utilisateur du premier instrument chirurgical et un utilisateur du second instrument chirurgical.

11. Procédé de détermination autonome de caractéristiques de dispositif chirurgical à activer pour une intervention chirurgicale, le procédé comprenant :
la détection d'un premier dispositif chirurgical (49045) dans l'environnement chirurgical, dans lequel le premier dispositif chirurgical (49045) comprend une première pluralité de caractéristiques, et dans lequel un concentrateur chirurgical (49095) comprend une pluralité de ressources ;
la détection d'un deuxième dispositif chirurgical (49090) dans l'environnement chirurgical, dans lequel le deuxième dispositif chirurgical (49090) comprend une deuxième pluralité de caractéristiques ;
la réception ou la déduction d'une indication de l'intervention chirurgicale à mettre en œuvre, l'intervention chirurgicale comprenant une pluralité de tâches chirurgicales ; et
la détermination d'une ou plusieurs caractéristiques de la première pluralité de caractéristiques pour le premier dispositif chirurgical (49045) à activer ou à désactiver pour mettre en œuvre une tâche chirurgicale de la pluralité de tâches chirurgicales et la détermination d'une ou plusieurs caractéristiques de la deuxième pluralité de caractéristiques pour le deuxième dispositif chirurgical (49090) à activer ou à désactiver pour mettre en œuvre une tâche chirurgicale de la pluralité de tâches chirurgicales,
dans lequel les caractéristiques sont des caractéristiques que le dispositif chirurgical respectif est capable de mettre en œuvre,
dans lequel une caractéristique activée est une caractéristique permettant de mettre en œuvre la tâche chirurgicale de la pluralité de tâches chirurgicales de manière autonome, et
dans lequel une caractéristique désactivée est une caractéristique permettant à un chirurgien de mettre en œuvre manuellement la tâche chirurgicale de la pluralité de tâches chirurgicales,
dans lequel la détermination est basée sur la pluralité de ressources.

12. Procédé selon la revendication 11, comprenant en outre :
l'envoi d'une indication de la ou des caractéristiques déterminées à un utilisateur du premier dispositif chirurgical (49045) et à un utilisateur du deuxième dispositif chirurgical (49090) ; ou
la transmission d'un signal au premier (49045) et/ou au deuxième (49090) dispositif pour activer la ou les caractéristiques déterminées.

13. Procédé selon la revendication 11, dans lequel le premier (49045) et/ou le deuxième (49090) dispositif est un dispositif de coupe endoscopique et la pluralité de premières et/ou deuxièmes caractéristiques comprend l'un ou plusieurs de ce qui suit : commande d'une source d'énergie, coupe, agrafage, orientation de bouton, orientation de corps, position de corps, serrage, force de mâchoire d'enclume, gestion de fente d'alignement de rechargement.

14. Procédé selon l'une quelconque des revendications 11 à 13, comprenant en outre la réception ou la détermination d'une indication de la première et de la deuxième pluralité de caractéristiques.

15. Procédé selon l'une quelconque des revendications 11 à 14, dans lequel la détection des premier (49045) et deuxième (49090) dispositifs comprend la détection du moment où les premier (49045) et deuxième (49090) dispositifs envoient une demande de couplage avec le concentrateur (49090), ou la détection du moment où ils ont été couplés au concentrateur (49095).

16. Procédé selon l'une quelconque des revendications 11 à 15, dans lequel le concentrateur chirurgical (49095) comprend une pluralité de ports, et dans lequel le premier dispositif chirurgical (49045) se lie au concentrateur chirurgical (49095) par l'intermédiaire d'un premier port de la pluralité de ports, et dans lequel le deuxième dispositif chirurgical (49090) se lie au concentrateur chirurgical (49095) par l'intermédiaire d'un second port de la pluralité de ports.

17. Procédé selon l'une quelconque des revendications 11 à 16, dans lequel la détermination des caractéristiques à activer comprend l'utilisation d'une optimisation associée à une ou plusieurs règles,
facultativement dans lequel l'optimisation associée à une ou plusieurs règles est basée sur l'un parmi : contexte chirurgical, type de la première pluralité de caractéristiques, type de la deuxième pluralité de caractéristiques, données historiques, ou la pluralité de ressources.

18. Procédé selon l'une quelconque des revendications 11 à 17, comprenant en outre :
la détection d'un troisième dispositif chirurgical dans l'environnement chirurgical, dans lequel le troisième dispositif chirurgical comprend une troisième pluralité de caractéristiques ; et
l'ajustement de la ou des caractéristiques de la première pluralité de caractéristiques à mettre en œuvre par le premier dispositif chirurgical (49045) et de la ou des caractéristiques de la deuxième pluralité de caractéristiques à mettre en œuvre par le deuxième dispositif chirurgical (49090) sur la base de la pluralité de ressources et de la troisième pluralité de caractéristiques,
facultativement dans lequel l'ajustement comprend la désactivation d'une caractéristique qui était précédemment activée.

19. Procédé selon l'une quelconque des revendications 11 à 18, comprenant en outre :
la détection d'un troisième dispositif chirurgical dans l'environnement chirurgical, dans lequel le troisième dispositif chirurgical comprend une troisième pluralité de caractéristiques ; et
la détermination d'une ou plusieurs caractéristiques de la troisième pluralité de caractéristiques à mettre en œuvre par le troisième dispositif chirurgical sur la base de la pluralité de ressources.

20. Procédé selon l'une quelconque revendication précédente, comprenant en outre :
la détermination d'une ou plusieurs caractéristiques facultatives de la première pluralité de caractéristiques à mettre en œuvre par le premier dispositif chirurgical et d'une ou plusieurs caractéristiques facultatives de la deuxième pluralité de caractéristiques à mettre en œuvre par le deuxième dispositif chirurgical sur la base de la pluralité de ressources,
facultativement dans lequel les caractéristiques facultatives sont sélectionnées par un utilisateur du premier instrument chirurgical et un utilisateur du second instrument chirurgical.

21. Programme informatique comprenant des instructions qui, lorsque le programme est exécuté par un ordinateur, amènent l'ordinateur à effectuer le procédé selon l'une quelconque des revendications 11 à 20.

22. Support lisible par ordinateur comprenant des instructions qui, lorsqu'elles sont exécutées par un ordinateur, amènent l'ordinateur à effectuer le procédé selon l'une quelconque des revendications 11 à 20.
